# FASCICULE DE BREVET EUROPEEN

(11) **EP 2 252 282 B1**
(45) Date de publication et mention de la délivrance du brevet: **15.07.2015**
(21) Numéro de dépôt: 08873234.2
(22) Date de dépôt: 31.12.2008
(51) Int. Cl.: A61K 31/22, A61K 31/663, A61P 17/00, A61P 43/00, A61K 8/49, A61K 8/55, A61K 31/33, A61K 31/66, A61Q 19/00, A61Q 19/08

(54) **COMPOSITION COSMETIQUE ET/OU DERMATOLOGIQUE COMPRENANT UN INHIBITEUR DE L'HMG-CoA REDUCTASE ET UN INHIBITEUR DE LA FARNESYL-PYROPHOSPHATE SYNTHASE**
KOSMETISCHE UND/ODER DERMATOLOGISCHE ZUSAMMENSETZUNG ENTHALTEND EINEN HMG-CoA REDUCTASE INHIBITOR UND EINEN FARNESYL-PYROPHOSPHATE SYNTHASE INHIBITOR
COSMETIC AND/OR DERMATOLOGICAL COMPOSITION COMPRISING AN HMG-CoA REDUCTASE INHIBITOR AND A FARNESYL-PYROPHOSPHATE SYNTHASE INHIBITOR

(30) Priorité: 03.01.2008 FR 0850019
(43) Date de publication de la demande: 24.11.2010
(73) Titulaire: Université d'Aix-Marseille, 13284 Marseille Cedex 07 (FR)
(72) Inventeur: CAU, Pierre, F-13540 Puyricard (FR); BOURGEOIS, Patrice, F-13127 Vitrolles (FR); BONNIOL, Vincent, F-13001 Marseille (FR); LEVY, Nicolas, F-13008 Marseille (FR)
(74) Mandataire: Novagraaf Technologies
(86) Numéro de dépôt international: PCT/FR2008/001843
(87) Numéro de publication internationale: WO 2009/112653

(56) Documents cités:
- EP-A- 1 127 573
- EP-A1- 1 566 177
- FONG L G ET AL: "A protein farnesyltransferase inhibitor ameliorates disease in a mouse model of progeria" SCIENCE, AMERICAN ASSOCIATION FOR THE ADVANCEMENT OF SCIENCE, US, WASHINGTON, DC, vol. 311, 17 mars 2006 (2006-03-17), pages 1621-1623, XP002420824 ISSN: 0036-8075 cité dans la demande
- GORDON L B ET AL: "Reduced adiponectin and HDL cholesterol without elevated C-reactive protein: Clues to the biology of premature atherosclerosis in Hutchinson-Gilford Progeria Syndrome" JOURNAL OF PEDIATRICS, MOSBY-YEAR BOOK, ST. LOUIS, MO, US, vol. 146, no. 3, 1 mars 2005 (2005-03-01), pages 336-341, XP004817122 ISSN: 0022-3476
- MALLAMPALLI ET AL.: "Inhibiting farnesylation reverses the nuclear morphology defect in a HeLa cell model for Hutchinson-Gilford progeria syndrome" PROCEEDINGS OF THE NATIONAL ACADEMY OF SCIENCE (PNAS), vol. 102, no. 40, 2005, pages 14416-14421, XP002495854
- RAMIREZ ET AL.: "Human progeroid syndromes, aging and cancer: new genetic and epigenetic insights into old questions" CELL. MOL. LIFE SCI., vol. 64, 2007, pages 155-170, XP019471731
- FONG L G ET AL: "A protein farnesyltransferase inhibitor ameliorates disease in a mouse model of progeria", SCIENCE, AMERICAN ASSOCIATION FOR THE ADVANCEMENT OF SCIENCE, WASHINGTON, DC; US, vol. 311, 17 March 2006 (2006-03-17), pages 1621-1623, XP002420824, ISSN: 0036-8075, DOI: 10.1126/SCIENCE.1124875

## Description

### Domaine technique

La présente invention se rapporte à une composition cosmétique et/ou dermatologique destinée au traitement de désordres cutanés et du système pileux.

Dans la description ci-dessous, les références entre parenthèses (X) renvoient à la liste de références à la fin des exemples.

### Art antérieur

Le noyau des cellules eucaryotes est délimité par une double membrane percée de pores, l'enveloppe nucléaire, qui contrôle les échanges moléculaires entre les deux compartiments nucléaire et cytoplasmique. Cette enveloppe isole partiellement le contenu du noyau, c'est-à-dire le matériel génétique et toute la machinerie enzymatique nécessaire aux fonctions du génome nucléaire.

L'enveloppe nucléaire est constituée de 2 membranes concentriques, la membrane externe, en continuité avec le réticulum endoplasmique, et la membrane interne. Cette dernière est bordée sur sa face interne par un maillage fibrillaire dense appelé *lamina* nucléaire. Il s'agit d'un réseau protéique essentiellement composé de polymères de lamines et de protéines associées. Chez les vertébrés, on distingue deux sous-classes de lamines : les lamines de type A (lamines A et C), et de type B (lamines B1, B2 et B3) qui participent toutes à l'élaboration de la *lamina.* Cette dernière est maintenue en place par l'association avec d'autres protéines, fixées à la membrane interne de l'enveloppe nucléaire (pour revue, Gruenbaum & al. 2005 **(19)).**

Les lamines sont des protéines en forme de filament appartenant à la famille des filaments intermédiaires (type V) qui ont toutes une structure commune : un court segment globulaire N-terminal (tête) séparé d'un autre segment globulaire C-terminal (queue) par un long domaine central organisé en plusieurs hélices *alpha (rod domain).* La queue globulaire contient notamment un signal de localisation nucléaire (*NLS*) permettant l'adressage au noyau après la synthèse. Le domaine central permet l'association de deux molécules de lamine parallèles et leur organisation en filaments par association des dimères "tête-bêche". Cette structure leur confère des propriétés mécaniques très résistantes.

Seules la lamine A et les lamines B subissent une maturation après la synthèse d'un précurseur (pour revue, Gruenbaum & al. 2000 **(20)).** La lamine C est directement synthétisée sous sa forme mature.

Le précurseur de la lamine A et des lamines B se termine par un motif *CaaX* caractéristique (*C* est une cystéine, a un acide aminé à chaîne aliphatique non chargée et *X* un acide aminé quelconque, ici une méthionine, pour revue, Lévy & Cau 2003 **(29)).**

Le motif *CaaX* C-terminal permet la fixation d'un acide gras (en général, un acide gras en C15, farnésyle) grâce à une farnésyl-transférase. Cette prénylation (le motif farnésyle dérive d'une unité aliphatique de base en C5 appelée isoprène) permet aux prélamines de venir s'insérer dans la membrane du réticulum endoplasmique après leur synthèse dans le cytosol. Elles y subissent l'action d'une endoprotéase elle-même insérée dans la membrane d'enveloppe du réticulum et dont le site actif est cytosolique. L'endoprotéase spécifique de la prélamine A est Face1 (ou ZMPSTE24, *Zinc Metallo-Profease* homologue de STE24 de levure), tandis que Face2 (ou Rce1, *ras-converting enzyme)* est spécifique des prélamines B. Ces enzymes catalysent l'hydrolyse de la liaison peptidique entre la cystéine et l'aminoacide suivant (aliphatique), raccourcissant les prélamines de 3 acides aminés. L'extrémité carboxyle de la cystéine farnésylée est ensuite reconnue par une isoprénylcystéine-carboxyméthyl transférase (ICMT) qui vient y fixer un groupement méthyle par estérification.

Seule la maturation de la prélamine A se poursuit avec un deuxième clivage endoprotéolytique par Face1, qui libère un farnésyl-peptide de 15 acides aminés et la lamine A mature. Cette lamine A, qui ne comporte plus l'acide gras, devient soluble, est importée dans le noyau grâce à son signal de localisation nucléaire, et se localise dans la *lamina* nucléaire elle-même ainsi que dans le reste du compartiment nucléaire, constituant un véritable squelette nucléaire (Gruenbaum & al. 2005 **(19)).** La lamine B mature en revanche possède toujours à l'extrémité C-terminale sa cystéine farnésylée et méthylestérifiée. Elle reste donc insérée dans la membrane d'enveloppe du réticulum, puis dans la face nucléoplasmique de l'enveloppe nucléaire, d'où sa localisation exclusive à la *lamina* nucléaire, sous la membrane interne de l'enveloppe nucléaire où elle est ancrée.

Sous le terme de prénylation on entend la fixation au groupe thiol d'une cystéine soit d'une chaîne farnésyle de 15 atomes de carbone, on parle alors de farnésylation, soit d'une chaîne géranyl-géranyle de 20 atomes de carbone, on parle alors de géranyl-géranylation (Reid & al. 2004 (39)), soit encore de tout autre dérivé de l'isoprène.

La farnésylation, catalysée par la farnésyl-transférase (FTase) qui reconnaît la séquence consensus C-terminale (*CaaX*), fixe préférentiellement un groupement farnésyle sur le résidu cystéine du motif.

La géranyl-géranylation est la fixation par la géranylgéranyl-transférase (GGTase) d'un groupement géranyl-géranyle sur le résidu cystéine du motif.

Ces acides gras sont issus de la voie de biosynthèse, qui, à partir de l'hydroxyméthyl-glutaryl-Coenzyme A, est utilisée par les cellules pour fabriquer notamment le cholestérol, les stéroïdes, l'hème de l'hémoglobine et les ubiquinones (Hampton & al. 1996 **(20)**).

La famille des protéines prénylées comporte environ 300 membres dans le génome humain, dont la majorité est identifiable par le motif C-terminal *CaaX* (Reid & al. 2004 **(39)**). Les protéines des familles *Ras, Rho, Rab* (Leung & al. 2006 **(28))** certaines protéines assurant une fonction d'import vers la mitochondrie (HDJ2), certaines protéines mitotiques (CENPE, CENPF) sont notamment prénylées (Winter-Vann & Casey 2005 **(51)**). En général, si dans le motif *CaaX* X est une sérine, une méthionine, une cystéine, une alanine ou un glutamate, l'isoprénoïde préférentiellement greffé est le farnésyle. Si X est une leucine, la reconnaissance du motif *CaaL* se fera préférentiellement par la GGTase, qui catalysera le transfert d'un groupement géranyl-géranyle (Basso & al. 2006 **(1)**). Il est probable que d'autres groupements dérivés de l'isoprène peuvent aussi être fixés sur cette cystéine, bien que cela ne soit pas décrit dans la littérature.

Chez l'homme, il existe trois gènes de lamines. Le gène *LMNA,* situé en 1q21.2-q21.3 (Wydner & al. 1996 **(52)**), donne par épissage alternatif les lamines A et C. Le gène *LMNA* est composé de 12 exons. Le début de l'exon 1 code l'extrémité globulaire N-terminale commune aux lamines A et C ; la fin de l'exon 1 et jusqu'au début de l'exon 7 codent la partie hélicoïdale centrale ; enfin, les autres exons codent l'extrémité globulaire C-terminale (Lévy & Cau 2003 **(29)**).

En fait, le gène code pour 4 produits épissés différemment, dont les lamines C et la prélamine A sont les 2 principaux (Lin & Worman 1993 (31)). La production différentielle des lamines A et C se fait par l'utilisation d'un site d'épissage alternatif au niveau de l'exon 10 du pré-messager, de telle sorte que la lamine C est codée par les exons de 1 à 10 et la lamine A est codée par les exons de 1 à 9, les 90 premières paires de bases de l'exon 10, et les exons 11 et 12 (lamine A-spécifiques).

Par conséquent, les peptides prélamine A et lamine C sont identiques au niveau des premiers 566 acides aminés, les extrémités C-terminales des lamines C et de la prélamine A contenant ensuite respectivement 6 et 98 acides aminés spécifiques.

Les lamines de type B comprennent trois protéines différentes (Shelton & al. 1981 **(43)**) : les lamines B1, B2 (les deux isoformes les plus représentées) et B3. Le gène *LMNB1* est situé en 5q23.3-q31.1 et comporte 11 exons codant la lamine B1 (Lin & Worman 1995 **(30)**). Le gène *LMNB2* est localisé en 19p13.3 et code pour les lamines B2 et B3 par un mécanisme d'épissage alternatif (Biamonti & al. 1992 **(2)**).

Les lamines de type B sont exprimées constitutivement dans toutes les cellules à partir des premiers stades de développement, tandis que les lamines de type A sont en général absentes dans les cellules souches embryonnaires (Stewart et al. 1987 **(45)**) et sont exprimées dans toutes les cellules somatiques différenciées. Leur expression est sujette à des régulations selon le tissu et au cours de la vie (Duque & al 2006 **(9)**). Il semble que leur expression ne soit pas nécessaire, puisque des souris chez lesquelles on a bloqué spécifiquement l'expression de lamine A, mais qui expriment tout de même la lamine C et les autres lamines, n'ont pas de phénotype apparent (Fong & al 2006 **(14)).**

Les lamines interagissent avec un nombre très élevé de partenaires protéiques ayant des fonctions très diverses; elles sont par conséquent impliquées dans un grand nombre de processus nucléaires, incluant la réplication et la réparation de l'ADN, le contrôle de la transcription et de l'épissage, l'organisation de la structure chromatinienne (pour revue, voir Shumaker & al. 2003 **(44),** Zastrow & al. 2004 **(54),** Hutchinson & al. 2004 **(26)**, Gruenbaum & al. 2005 **(19)**). Les altérations de la structure de la *lamina* sont à l'origine de nombreuses pathologies héréditaires humaines. Elles sont dues à des mutations des gènes codant les lamines, ou d'autres protéines de la *lamina.* On a regroupé ces pathologies sous le terme générique de laminopathies (Broers & al. 2006 **(5)**, Mattout & al 2006 **(33)**). Récemment, des mutations dans les gènes des enzymes responsables de la maturation des lamines (Face1 notamment), ont été identifiées, donnant lieu à des pathologies appartenant également au groupe des laminopathies (Navarro & al 2004 **(36)** et 2005 **(35)**).

A ce jour, la seule pathologie chez l'homme associée à des mutations des gènes *LMNB1* ou 2 est une leucodystrophie causée par une duplication complète du gène *LMNB1* (Padiath & al 2006 **(37)**). Un doute subsiste sur l'implication potentielle de variations de séquences trouvées dans *LMNB2* chez des patients atteints du syndrome de Barraquer-Simon (Hegele & al 2006 **(22)**). Cependant, il a été démontré *in vitro* par des expériences de RNAi (RNA-interférence) (Harborth & al. 2001 **(21)**), ainsi que chez le modèle murin (Vergnes & al. 2004 **(50)**, que les lamines de type B sont essentielles pour le développement et l'intégrité cellulaire. En effet, la déficience en lamine B1 entraîne chez la souris une létalité périnatale. Par ailleurs, les noyaux des fibroblastes embryonnaires des mêmes souris *LMNB1* déficientes montrent des altérations remarquables de la morphologie nucléaire, proches de celles observées chez les patients porteurs de mutations du gène *LMNA.* De plus, il a été montré récemment que les lamines B sont nécessaires à la formation du fuseau de division pendant la mitose, ce qui tend à prouver que leur rôle est dynamique et multiple au cours du cycle cellulaire, et pas uniquement restreint au maintien de l'architecture du noyau (Tsai & al. 2006 **(48)**). Sur ce dernier rôle, un article récent constitue la démonstration de la fonction structurale des lamines B : des cellules artificiellement privées de lamines B1 ont un noyau "flottant" dans la cellule, qui tourne sur lui-même (Liu & al. 2007 **(45)**). La redondance fonctionnelle existant entre les deux lamines B1 et B2 est sans doute aussi le reflet direct de leur indispensabilité, exerçant une forte pression de sélection et masquant l'effet de mutations éventuelles de la séquence des gènes correspondants.

Les altérations fonctionnelles des lamines A/C, dues à des mutations du gène *LMNA,* sont à l'origine d'au moins 15 désordres regroupant des pathologies très diverses dans un spectre clinique allant de formes peu sévères, affectant un seul tissu de façon isolée, à des formes systémiques létales en période périnatale.

Nombre de mutations du gène *LMNA* modifient de façon notable l'assemblage des protéines dans l'enveloppe nucléaire et en perturbent le fonctionnement. Dans les cellules de divers tissus, la morphologie des noyaux est altérée : ils présentent souvent des hernies qui extrudent du matériel génétique dans le cytoplasme (Goldman & al. 2004 **(18)**).

Les protéines habituellement associées à l'enveloppe nucléaire, les lamines B, certaines protéines des pores nucléaires et les protéines LAP2, sont absentes du pourtour de ces hernies.

Ces anomalies morphologiques sont suivies d'altérations fonctionnelles, qui finissent par entraîner la mort cellulaire. Parmi l'ensemble des pathologies regroupées sous la dénomination de laminopathies, seules celles liées à l'accumulation anormale d'une forme prénylée de protéine sont concernées par la présente invention.

Ce sont principalement le syndrome de Hutchinson-Gilford, ou Progéria (De Sandre-Giovannoli & al. 2003 **(7)**, Eriksson & al. 2003 **(11)**), et la dermopathie restrictive (Navarro & al. 2004 **(36)**). Dans ces 2 syndromes, la cause physiopathologique est une accumulation et une persistance dans les cellules des malades de prélamine farnésylée non maturée.

La dermopathie restrictive, létale autour de la période natale, se caractérise par des signes cliniques qui sont presque tous la conséquence d'un déficit cutané qui restreint les mouvements *in utero.* Cette pathologie est très rare. La peau est rigide et tendue, elle cède par endroits, provoquant par exemple des déchirures au niveau des aisselles ou du cou. Les cils, les sourcils et le duvet cutané sont absents ou très clairsemés. Un hydramnios est souvent présent, et la diminution des mouvements foetaux est signalée dès le 6ème mois de grossesse. Au niveau du squelette, la radiographie révèle des contractures de toutes les articulations, des pieds en piolet, des clavicules fines, dysplasiques et bi-partites, des côtes en ruban, des os longs tubulaires des bras et une déminéralisation au niveau du crâne. La transmission de la dermopathie restrictive létale est récessive autosomique.

Des mutations de LMNA et de ZMPSTE24/Face1 ont été rapportées pour cette pathologie (Navarro & al. 2004 **(36)**). Dans les 2 cas, le mécanisme physiopathologique est le même : la prélamine A ne peut pas maturer (mutation nulle de Face1 ou disparition du site de clivage par mutation de la prélamine A), et reste farnésylée, et donc insérée dans la membrane nucléaire. L'accumulation et la persistance dans les cellules de ces précurseurs anormaux, qui empêchent probablement l'interaction normale des lamines B et C avec leurs partenaires, entraîne la mort des cellules et, à brève échéance, du patient. Il a été clairement démontré que c'est bien la persistance du groupement farnésyle, et non l'absence de lamine A mature, comme on pourrait le penser de prime abord, qui est responsable de la toxicité cellulaire (Fong & al. 2004 **(16)**).

En avril 2003, à partir d'un recoupement des symptômes communs à la dysplasie acromandibulaire et à certaines maladies se traduisant par un vieillissement prématuré, les inventeurs ont montré que la Progéria, la forme la plus typique et la plus grave de vieillissement précoce, résulte d'une mutation du gène *LMNA* (De Sandre-Giovannoli & al. 2003 **(7)**). Les enfants atteints par cette maladie, aussi nommée syndrome de Hutchinson-Gilford, souffrent d'un vieillissement accéléré, jusqu'à dix fois plus rapide que celui d'un individu normal, et ont une espérance de vie qui ne dépasse pas 13 ans. En Europe, un enfant sur environ six millions est touché. Les symptômes sont un vieillissement cutané, une calvitie, une réduction de la taille de la mâchoire et des problèmes liés à la vieillesse, par exemple, une raideur des articulations et des troubles cardio-vasculaires. Ces derniers, tels l'infarctus du myocarde ou l'athérosclérose, sont souvent la cause de la mort.

La mutation incriminée, située dans l'exon 11 du gène *LMNA,* active un site cryptique d'épissage du pré-ARNm, conduisant à un ARNm délété de 150 nucléotides (De Sandre-Giovannoli & al. 2003 **(7)**, Eriksson & al. 2003 **(11)**). Cet ARNm délété est traduit en une prélamine A anormale, la progérine, qui ne peut pas être maturée en lamine A normale : l'absence de 50 acides aminés de l'exon 11 comportant le site de reconnaissance de la protéase bloque le 2^{e} clivage de la progérine dont l'extrémité C-terminale conserve son groupement farnésyle. Elle reste donc insérée dans la face nucléoplasmique de l'enveloppe nucléaire, qui présente les altérations caractéristiques, hernies du nucléoplasme dans le cytosol et anomalies de la répartition de l'hétérochromatine périphérique (Goldman & al. 2004 **(18)**). Ici encore, c'est la persistance du groupe farnésyle, par ailleurs nécessaire pour l'ancrage à la membrane d'enveloppe du réticulum dans laquelle sont localisées certaines des enzymes responsables de la maturation (clivages, méthylation) qui est responsable de la toxicité cellulaire de la progérine (Fong & al. 2004 **(16)**).

Ces pathologies systémiques présentent la particularité d'être associées à l'apparition précoce de signes habituellement liés au vieillissement. Leur caractéristique physiopathologique commune est de générer une lamine prénylée, avec les conséquences décrites.

Deux études récentes ont montré qu'une réduction de l'accumulation intranucléaire de la prélamine farnésylée, tronquée ou non, prévient efficacement l'apparition du phénotype cellulaire. La première a été réalisée sur le modèle murin progéroïde déficient en protéase Face1 (Pendas & al. 2002 **(38)**). Lorsqu'elles sont croisées avec des souris exprimant moitié moins de Lamine A (souris *Lmna* +/-), les effets de l'absence de Face1 sont amoindris (Varela & al. 2005 **(49)**). La deuxième étude montre que le traitement de cellules de patients HGPS par des *morpholino* (oligonucléotides antisens) ciblant le site d'épissage cryptique abolit le phénotype mutant (Scaffidi & Mistelli 2005 **(43)).**

Plusieurs études récentes (voir Scaffidi & Mistelli 2006 **(42)**) montrent l'implication de la lamine A dans le processus de vieillissement physiologique. En particulier, il a été démontré qu'au cours du vieillissement physiologique, la progérine est synthétisée par les cellules en l'absence de toute mutation du gène *LMNA* en raison de l'utilisation à bas bruit du site cryptique d'épissage de l'exon 11. Cette progérine se localise dans la *lamina,* à la périphérie du noyau des cellules. Le noyau de cellules de patients âgés «normaux» peut présenter des hernies caractéristiques d'une laminopathie causée par ces événements d'épissage accidentel, qui entraînent des anomalies des fonctions cellulaires et sont probablement au moins en partie responsables de leur vieillissement.

Dans la peau *in vivo,* la progérine est aussi synthétisée par une sous-population de fibroblastes dermiques et de kératinocytes, cellules dans lesquelles elle s'accumule avec l'âge. La progérine pourrait donc être un marqueur du vieillissement cutané (McClintock et al. 2007 **(34)**).

Il apparaît que des mécanismes moléculaires identiques sont d'une part responsables des signes de vieillissement prématuré des individus atteint de Progéria et d'autre part, à un niveau beaucoup plus faible, interviennent dans le vieillissement physiologique d'individus non porteurs de mutations.

Le document Ramirez et al. « human progeroid syndromes aging and cancer : new genetic and epigenetic insights into old question » Cell. Mol. Life Sci, vol. 64, 2007, page 155-170 suggère l'utilisation de statins et d'aminobisphosphonates afin de bloquer la farnésylation aberrante de la lamine A en agissant sur la voie de synthèse du farnésyl pyrophosphate.

Le document brevet EP 1566177 décrit l'incubation de culture de fibroblastes avec une composition comprenant du pamidronate ou de l'alendronate qui permet de stimuler la synthèse de collagène 1 par lesdits fibroblastes (exemples 1 à 3). Ce document décrit également une composition comprenant du pamidronate ou de l'alendronate qui permet de réduire les rides (exemple 5).

Il existe dans l'art antérieur deux approches thérapeutiques décrites pour améliorer le phénotype cellulaire causé par la production pathologique de progérine. La première de ces solutions est tout simplement d'empêcher l'utilisation par le spliceosome de ce site d'épissage cryptique dans l'exon 11, en le "masquant" par traitement avec un oligonucléotide antisens (Scaffidi & Misteli 2005 **(41)**), ou avec un rétrovirus produisant un siARN (Huang & al. 2005 **(25)**). Les résultats sont prometteurs *in vitro,* mais il s'agit ici de thérapie "génique", et le développement d'un médicament autour de cette approche est immanquablement long et compliqué, avec tous les inconvénients liés à la vectorisation des OAS pour obtenir un effet *in vivo.* La deuxième solution consiste à inhiber la farnésyl-transférase, l'enzyme qui catalyse le transfert du groupement farnésyle sur les prélamines à partir de farnésyle-pyrophosphate. Lorsque de tels inhibiteurs (FTI) sont utilisés, une enveloppe nucléaire «normale» n'est que partiellement restaurée sur des cellules HGPS (Progéria) en culture, et la survie de souris RD (KO ZMPSTE24) est améliorée (Glynn & Glover 2005 **(17)**, Capell & al. 2005 **(6**)**,** Toth & al. 2005 **(47)**, Fong & al. 2006 **(15)).**

Cependant, le blocage de la farnésylation peut induire une géranyl-géranylation compensatoire (Bishop & al. 2003 **(3)**, Varela & al. 2008 **(55)).**

D'autre part, il a été rapporté récemment que les FTI induisent un arrêt du cycle cellulaire en bloquant le protéasome (Demyanets & al. 2006 (8), Efuet & Keyomarsi 2006 **(10)**). Ainsi, le traitement induit sans doute une accumulation dans le nucléoplasme de progérine probablement ubiquitinylée, non dégradée par le protéasome.

Par ailleurs, des travaux récents rapportent que la diminution du taux de farnésylation de la progérine *in vivo* est très faible, de l'ordre de 5 % (Young & al. 2006 **(53)**), ce qui ne suffit pas à expliquer la restauration de la morphologie nucléaire observée *in vitro.*

Finalement, les FTI sont spécifiques d'une seule des voies de prénylation protéique, et ne peuvent être envisagés comme inhibiteurs globaux des prénylations post-traductionnelles.

Par ailleurs, il est rapporté que l'absence totale d'une des enzymes de cette voie, la mévalonate-kinase, est létale durant l'enfance (mutation homozygote perte de fonction du gène codant pour cette enzyme, syndrome rapporté par Hoffmann & al. 2003 **(24)**).

### Exposé de l'invention

Après de longues recherches, les inventeurs ont montré que l'association d'un inhibiteur de l'hydroxyméthylglutaryl-coenzyme A réductase (famille des statines) et d'un inhibiteur de la farnésyl-pyrophosphate synthase (famille des amino-biphosphonates, NBP), ou de l'un de leurs sels physiologiquement acceptable, est un traitement efficace des situations, pathologiques ou non, liées à l'accumulation et/ou la persistance dans les cellules de protéines prénylées, en ce sens qu'elle agit sur l'ensemble de la voie de prénylation des protéines, tant en C15 qu'en C20 ou dans les formes non caractérisées. Les inventeurs ont en outre constaté que l'association d'un inhibiteur de l'hydroxyméthylglutaryl-coenzyme A (HMG-CoA) réductase et d'un inhibiteur de la farnésyl-pyrophosphate synthase présente un effet synergique dans la restauration du phénotype normal chez des fibroblastes de patients atteints de Progéria. L'effet de l'association est nettement supérieur à ce qu'est l'effet de l'un ou l'autre des inhibiteurs utilisé individuellement.

L'utilisation de l'association sur des cellules de patients atteints de Progéria et dans un modèle de souris reproduisant la dermopathie restrictive conduit à une inhibition de la prénylation des protéines, donc à l'apparition de prélamine A non farnésylée et à l'amélioration des symptômes nucléaires (Varela & al. 2008 **(24)**).

La présente se rapporte donc à l'utilisation d'au moins un inhibiteur de l'hydroxyméthylglutaryl-coenzyme A (HMG-CoA) réductase et d'au moins un inhibiteur de la farnésyl-pyrophosphate synthase, ou de l'un de leurs sels physiologiquement acceptable, dans la préparation d'une composition, cosmétique et/ou dermatologique. Cette composition peut être destinée par exemple au traitement des situations, pathologiques ou non, liées à l'accumulation et/ou la persistance dans les cellules de protéines prénylées.

En particulier, la présente invention se rapporte à une composition topique dermatologique comprenant :
- au moins un inhibiteur de l'hydroxyméthylglutaryl-coenzyme A (HMG-CoA) réductase, et
- au moins un inhibiteur de la farnésyl-pyrophosphate synthase,
pour son utilisation dans le traitement de désordres cutanés et/ou pileux choisis dans le groupe comprenant le vieillissement cutané précoce pathologique et/ou le vieillissement myo-lipo-cutané précoce pathologique.

La présente invention se rapporte également a l'utilisation cosmétique non thérapeutique d'une composition cosmétique comprenant :
- au moins un inhibiteur de l'hydroxyméthylglutaryl-coenzyme A (HMG-CoA) réductase, et
- au moins un inhibiteur de la farnésyl-pyrophosphate synthase pour le traitement de désordres cutanés et/ou pileux choisis dans le groupe comprenant le vieillissement cutané et/ou la lipodystrophie.

Selon un autre aspect, la présente décrit également l'utilisation
- Conjointe ou simultanée, c'est-à-dire que les au moins deux produits suivants (l'au moins un inhibiteur de l'hydroxyméthylglutaryl-coenzyme A réductase d'une part et l'au moins un inhibiteur de la farnésyl-pyrophosphate synthase) sont administrés dans une seule composition les contenant,
- concomitante, c'est-à-dire de manière séparée, chacun des au moins deux produits suivants (l'au moins un inhibiteur de l'hydroxyméthylglutaryl-coenzyme A réductase d'une part et l'au moins un inhibiteur de la farnésyl-pyrophosphate synthase) étant administrés de manière indépendante par des voies d'administration identiques ou différentes, l'administration de ces deux produits étant réalisée de manière concomitante, ou
- successive, c'est-à-dire de manière séparée, chacun des au moins deux produits suivants (l'au moins un inhibiteur de l'hydroxyméthylglutaryl-coenzyme A réductase d'une part et l'au moins un inhibiteur de la farnésyl-pyrophosphate synthase) étant administrés de manière indépendante par des voies d'administration identiques ou différentes, l'administration de ces deux produits étant réalisée de manière successive, un produit après l'autre,
d'au moins un inhibiteur de l'hydroxyméthylglutaryl-coenzyme A (HMG-CoA) réductase et d'au moins un inhibiteur de la farnésyl-pyrophosphate synthase, dans le traitement de désordres cutanés et/ou pileux.

Ainsi, selon cet aspect, les composés précités peuvent être utilisés en mélange pour une administration simultanée ou séparés pour une administration concomitante ou successive.

Par exemple, le, au moins un, inhibiteur de Phydroxyméthylglutaryl-coenzyme A (HMG-CoA) réductase peut être administré en premier, puis le, au moins un, inhibiteur de la farnésyl-pyrophosphate synthase peut être administré en second. Dans un autre exemple, le, au moins un, inhibiteur de la farnésyl-pyrophosphate synthase peut être administré en premier, puis le, au moins un, inhibiteur de l'hydroxyméthylglutaryl-coenzyme A (HMG-CoA) réductase peut être administré en second.

Selon un troisième exemple, le, au moins un, inhibiteur de la farnésyl-pyrophosphate synthase d'une part et le, au moins un, inhibiteur de l'hydroxyméthylglutaryl-coenzyme A (HMG-CoA) réductase, d'autre part, sont administrés au même moment, mais, par exemple, par des voies d'administration différentes, par exemple choisies parmi une administration orale, topique ou par injection).

Quel que soit le mode de réalisation de la présente invention, notamment dans les aspects précités,

Par exemple, la composition utilisée dans la présente invention peut être préparée extratemporanée au moment ou juste avant l'administration. La préparation peut être réalisée, par exemple, au moyen d'une seringue ou d'un système de pompe qui permet de mélanger les deux composés précités au moment de l'administration ou juste avant. L'administration peut être réalisée par exemple au moyen d'une seringue double et/ou d'une pompe cosmétique, par exemple au moyen d'une pompe commercialisée par la société Kemai (marque de commerce).

En d'autres termes, même si dans la présente description il est fait référence à une composition, on décrit également que chacun des composés de la composition peut être administré conjointement ou concomitamment aux autres composés (par exemple dans une seule composition ou dans deux compositions ou dans trois compositions, chacune de ces compositions comprenant un ou plusieurs des composants précités, le mode d'administration de chacun des composés ou composition pouvant être identique ou différent), ou indépendamment les uns des autres, par exemple successivement, par exemple administration indépendante d'un inhibiteur de l'hydroxyméthylglutaryl-coenzyme A (HMG-CoA) réductase, administration indépendante d'au moins un inhibiteur de la farnésyl-pyrophosphate synthase, ces administrations étant réalisées sur un même patient, conjointement, concomitamment ou successivement ou alternativement, dans un ordre qui est celui précité ou un autre ordre. Ces différentes administrations peuvent être réalisées, indépendamment l'une de l'autre ou de manière liée (composition ou co-administration), par un mode d'administration identique ou différent (injection, ingestion, application topique, etc.), une ou plusieurs fois par jour, pendant un ou plusieurs jours successifs ou non.

Cette composition peut être destinée par exemple au traitement des situations, pathologiques ou non, liées à l'accumulation et/ou la persistance dans les cellules de protéines prénylées.

Il est aussi dans la portée de l'invention d'utiliser des composés qui sont à la fois des inhibiteurs de l'hydroxyméthylglutaryl-coenzyme A (HMG-CoA) réductase et des inhibiteurs de la farnésyl-pyrophosphate synthase.

Dans la présente la composition cosmétique et/ou dermatologique décrite peut être destinée au traitement des situations, pathologiques ou non, liées à l'accumulation et/ou la persistance dans les cellules de progérine ; encore plus particulièrement, au traitement des situations liées à l'accumulation et/ou la persistance dans les cellules de prélamine A farnésylée, que celle-ci soit ou non tronquée ou modifiée.

Notamment, puisqu'il est admis que le vieillissement physiologique est notamment une conséquence de la présence de progérine dans les cellules au cours de la vie. La progérine se concentre en particulier dans les cellules mésenchymateuses, la composition peut être destinée à prévenir les effets du vieillissement cellulaire, particulièrement au niveau de la peau et/ou de l'endothélium vasculaire.

Une composition topique dermatologique comprenant :
- au moins un inhibiteur de l'hydroxyméthylglutaryl-coenzyme A (HMG-CoA) réductase, et
- au moins un inhibiteur de la farnésyl-pyrophosphate synthase, pour son utilisation dans le traitement de désordres cutanés et/ou pileux choisis dans le groupe comprenant le vieillissement cutané précoce pathologique et/ou le vieillissement myo-lipo-cutané précoce pathologique.

Par exemple, ladite composition cosmétique est utilisée afin de prévenir et/ou de traiter des désordres cutanés et/ou la lypodystrophie. Ces désordres cutanés peuvent être des désordres d'origine naturelle, pathologique ou liés à un traitement thérapeutique. Les désordres d'origine naturelle sont ceux liés au vieillissement naturel, par exemple le vieillissement cutané. Les désordres pathologiques sont ceux liés par exemple au vieillissement cutané hormonal, au vieillissement photo-induit, au vieillissement cutané précoce, au vieillissement myo-lipo-cutané, à la lipodystrophie, dermopathie restrictive. Les désordres liés à un traitement sont ceux liés par exemple aux traitements chimiothérapeutiques, aux irradiations par les rayons X, les rayons gamma, les ultraviolets ou ceux liés aux effets secondaires des traitements.

La Progéria s'accompagne d'une alopécie très marquée. À partir de 2 ans, les enfants atteints perdent leurs cheveux, qui deviennent épars, blancs et soyeux. Les cils et les sourcils tombent aussi, et les poils du corps sont rares ou complètement absents (voir la description détaillée des symptômes cliniques faites par R. Hennekam (2006). Cette alopécie est également observée sur le modèle murin de la progéria (souris déficientes pour *Zmpste24*). Le traitement avec une association de pravastatine et de zolédronate conforme à la présente invention restaure de manière tout à fait inattendue au moins partiellement le pelage de ces souris.

La composition cosmétique et/ou dermatologique de la présente invention peut être destinée au traitement de tout être vivant, homme ou animal, particulièrement pour la prévention des effets du vieillissement cellulaire. La composition trouve donc une application aussi bien en médecine humaine, en médecine vétérinaire, en cosmétique et/ou en dermatologie. Elle permet par exemple de rétablir et/ou prévenir des désordres cutanés, physiologiques ou non, incluant le vieillissement de la peau et l'altération ou la disparition du système pileux.

Selon l'invention, tout inhibiteur de la farnésyl-pyrophosphate synthase, ou l'un de ses sels physiologiquement acceptables, peut être utilisé dans la préparation de la composition selon l'invention.

Les sels physiologiquement acceptables peuvent être par exemple des sels formés avec les acides chlorhydrique, bromhydrique, nitrique, sulfurique, phosphorique, les acides carboxyliques tels que par exemple les acides acétique, formique, propionique, benzoïque, maléique, fumarique, succinique, tartrique, citrique, oxalique, glyoxylique, aspartique, alcanes sulfoniques tels que les acides méthane ou éthane sulfoniques, arylsulfoniques tels que les acides benzène ou paratoluène sulfoniques.

Particulièrement l'inhibiteur de la farnésyl-pyrophosphate synthase peut être un des membres de la famille des polyphosphonates, particulièrement des aminobiphosphonates (NBP), ou l'un de ses sels physiologiquement acceptables.

Les polyphosphonates sont des molécules synthétiques très utilisées dans le traitement de l'ostéoporose et de la régénération osseuse.

Le terme de phosphonate s'applique à des molécules très semblables au phosphate :

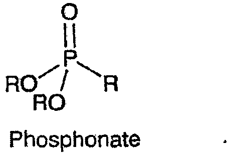

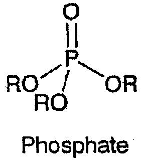

Le coeur des biphosphonates (BP) est l'équivalent d'une liaison P-O-P comme dans l'ATP par exemple, mais où l'oxygène est remplacé par un carbone. Ceci confère une stabilité tout à fait particulière à ces molécules.

Un biphosphonate simple serait équivalent à l'ADP, les 2 groupes phosphates (O₃P-) étant remplacés par le groupe biphosphonate.

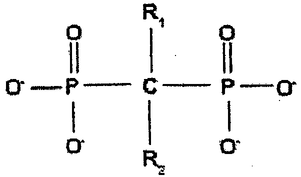

Le carbone central, à la différence de l'oxygène des phosphates, peut encore être impliqué dans 2 liaisons, et c'est la nature des groupements greffés sur ce carbone qui fait la spécificité des biphosphonates.

Lorsque les chaînes "latérales" (R1 et R2) comportent une fonction amine (NH), ou plus généralement un ou plusieurs atomes d'azote, on parle d'amino-biphosphonate, ou NBP.

Bien sûr, d'autres substituants peuvent être fixés sur les oxygènes.

L'acide pyrophosphorique, ou pyrophosphate en solution (PPi) 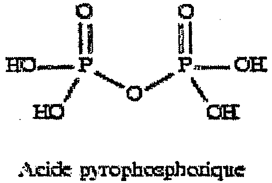 est utilisé dans de nombreuses réactions métaboliques comme transporteur de substrats, et il est restitué à la fin de la réaction. L'une des voies métaboliques utilisant des molécules couplées au pyrophosphate est celle de la prénylation protéique.

Le greffage d'un isopentènyl-PP (unité de base en C5) sur un géranyl-PP (C10) pour donner du farnésyl-PP, réaction catalysée par l'enzyme farnésyl-pyrophosphate synthase (FPS), libère un PPi.

C'est cette étape qui est spécifiquement inhibée par les NBP.

À cet égard et à titre d'exemple, l'aminobiphosphonate (inhibiteur de la farnésyl-pyrophosphate synthase) peut être choisi parmi
- l'acide alendronique ou sa forme ionique, l'alendronate ;
- l'acide clodronique ou sa forme ionique, le clodronate ;
- l'acide étidronique ou sa forme ionique, l'étidronate ;
- l'acide ibandronique, ou sa forme ionique, l'ibandronate ;
- l'acide médronique ou sa forme ionique le médronate ;
- l'acide néridronique ou sa forme ionique, le néridronate ;
- l'acide olpadronique ou sa forme ionique, l'olpadronate ;
- l'acide pamidronique ou sa forme ionique, le pamidronate ;
- l'acide risédronique ou sa forme ionique, le risédronate ;
- l'acide tiludronique ou sa forme ionique le tiludronate;
- l'acide zolédronique ou sa forme ionique le zolédronate ;
- l'acide 4-N,N-diméthylaminométhane diphosphonique ou sa forme ionique le diméthylaminométhanediphosphonate ;
- l'α-amino-(4-hydroxybenzylidène) diphosphonate.

Préférentiellement selon l'invention, on préfère utiliser l'acide zolédronique (aussi appelé acide zolendronique) ou sa forme ionique, le zolédronate (aussi appelé zolendronate).

Selon l'invention, tout inhibiteur de HMG-CoA réductase, ou l'un de ses sels physiologiquement acceptables, peut être utilisé dans la préparation de la composition.

Particulièrement l'inhibiteur de HMG-CoA réductase peut être une molécule de la famille des statines, qu'elle soit liposoluble ou hydrosoluble, ou l'un de ses sels physiologiquement acceptables.

Les statines ont été mises en évidence chez des champignons. Elles ont une activité inhibitrice de l'HMG-CoA réductase, enzyme clé de la biosynthèse du cholestérol et des stéroïdes, qui catalyse la réduction de l'hydroxyméthyl-glutarate couplé au Coenzyme A en acide mévalonique (mévalonate en solution). Cette inhibition est assurée par leur analogie structurale avec le squelette de l'hydroxyméthylglutarate. La voie métabolique impliquée est certes celle de la biosynthèse du cholestérol, mais c'est aussi celle de la synthèse des groupes prényles, des polymères de l'unité de base à 5 carbones isoprène utilisés pour modifier environ 300 protéines dans les cellules et leur attacher une queue lipophile, permettant notamment leur ancrage dans les membranes.

Les principaux polyprènes, tous issus du pyruvate et de l'HMG-CoA, sont le géranyle (C10), le farnésyle (C15) et le géranyl-géranyle (C20).

Toutes les statines sont globalement hépatosélectives, mais toutes n'ont pas le même mode d'entrée dans les cellules. En effet, pravastatine et rosuvastatine sont toutes deux hydrophiles, donc hydrosolubles, au contraire de toutes les autres qui sont lipophiles, donc peuvent diffuser librement à travers les membranes plasmiques (bicouches lipidiques), ce qui explique sans doute leur plus haute toxicité. Les statines hydrosolubles ont besoin d'un transporteur spécifique pour entrer dans la cellule, *Organic Anion Transporter 3,* ou OAT3, ouSLC22A8 (Takedaa & al. 2004 **(46)**).

Elles sont très utilisées pour le traitement de l'hypercholestérolémie, et leurs effets secondaires, rares, sont bien caractérisés. Ce sont notamment des cas de rhabdomyolyse (1 à 7% des cas selon la molécule utilisée, Evans & al. 2002 **(12)**), dont le signe avant-coureur, des douleurs musculaires chez le patient traité, entraîne l'arrêt immédiat du traitement.

A cet égard et à titre d'exemple, une statine peut être choisie parmi l'atorvastatine, la simvastatine, la pravastatine, la rivastatine, la mévastatine (ou compactine), la fluindostatine, la vélostatine, la fluvastatine, la dalvastatine, la cérivastatine, la pentostatine, la rosuvastatine, la lovastatine, la pitavastatine, ou de l'un de leurs sels physiologiquement acceptables.

La lovastatine, la pravastatine et la simvastatine sont les molécules dérivées de métabolites fongiques, tandis que les autres, (atorvastatine, cérivastatine, fluvastatine, pitavastatine et rosuvastatine) sont entièrement synthétiques. Préférentiellement selon l'invention, I utilise la pravastatine, une statine semi-naturelle hydrosoluble.

Bien entendu il est possible selon l'invention d'utiliser un, deux ou plusieurs inhibiteur(s) de la farnésyl-pyrophosphate synthase associés à un, deux ou plusieurs inhibiteur(s) de HMG-CoA réductase.

La composition décrite peut être destinée au traitement de situations, pathologiques ou non, nécessitant d'inhiber la prénylation des protéines. Ces pathologies peuvent être étiquetées ou non, par exemple le syndrome de Noonan, le syndrome cardio-fascio-cutané, ou les maladies liées à une prénylation anormale ou persistante de Ras et des protéines de transduction du signal.

Dans la présente la composition destinée au traitement de situations, pathologiques ou non, présentant des signes de vieillissement, qu'il soit naturel, prématuré ou accéléré notamment en cas de signes de détérioration de l'endothélium vasculaire (protection de l'endothélium vasculaire), de vieillissement de la peau, de perte de poils et/ou de cheveux, et de lyse osseuse est décrite.

Dans la présente une composition dermatologique et/ou cosmétique destinée au traitement de la Progéria (HGPS, Hutchinson-Gilford Progeria Syndrome) et de la dermopathie restrictive (DR ou RD) est décrite.

Selon l'invention, l'inhibiteur de la farnésyl-pyrophosphate synthase et l'inhibiteur de la HMG-CoA réductase sont avantageusement présents dans la composition à des doses physiologiquement efficaces.

De manière générale, les quantités à administrer peuvent être adaptées en fonction du patient, de la pathologie, du mode d'administration, etc. Il est entendu que des applications répétées peuvent être réalisées, éventuellement en combinaison avec d'autres ingrédients actifs ou tout véhicule.

En général la dose journalière des inhibiteurs sera la dose minimum pour obtenir l'effet dermatologique et/ou cosmétique souhaité.

Selon l'invention, l'inhibiteur de l'hydroxyméthylglutaryl-coenzyme A (HMG-CoA) réductase et l'inhibiteur de la farnésyl-pyrophosphate synthase, peuvent être utilisés dans la composition, en mélange avec un ou plusieurs excipients ou véhicules inertes, c'est-à-dire physiologiquement inactifs et non toxiques. On peut citer par exemple les ingrédients habituellement utilisés dans les préparations cosmétiques ou dermatologique.

La composition cosmétique et/ou dermatologique de la présente invention comprend au moins un produit cosmétique et/ou dermatologique. Par « produit cosmétique et/ou dermatologique » on entend tout produit qui permet de constituer, avec les deux inhibiteurs précités, une composition permettant une application cosmétique et/ou dermatologique, c'est-à-dire une composition pouvant être appliquée sur la peau ou permettant de traiter la peau du point de vue cosmétique et/ou dermatologique.

Au minimum, ce produit cosmétique et/ou dermatologique est celui permettant de mettre en suspension ou de solubiliser les deux inhibiteurs afin de pouvoir les appliquer sur la peau. Par exemple, dans ce cas, il peut s'agir d'alcool, d'eau, d'un tampon, d'une solution saline, d'une solution isotonique, ou d'un mélange de deux ou plusieurs de ceux-ci.

De préférence, bien entendu, le produit cosmétique et/ou dermatologique est un produit acceptable sur le plan physiologique, en particulier pour une application topique.

Le produit cosmétique et/ou dermatologique utilisable dans la composition de la présente invention peut également comprendre ou être constitué de tout composé ou mélange de composés utilisé(s) couramment dans les compositions cosmétiques et/ou dermatologiques.

Il peut s'agir par exemple d'un ou plusieurs des composés choisi(s) dans le groupe comprenant les tensioactifs, les agents épaississants, les agents gélifiants, les conservateurs, les agents humectants, les agents émulsifiants, du parfum, les silicones, les agents chélatants, les antioxydants, les colorants cosmétiques, les agents fongicides, les agents antibactériens, les agents filmogènes, les agents stabilisateurs, les agents tampons, les filtres UV, les liants, les stabilisateurs d'émulsions.

Par « agent émulsifiant », on entend tout composé permettant de favoriser les mélanges de liquides non miscibles. Ces composés sont connus de l'homme du métier. Par exemple, l'agent émulsifiant peut être choisi dans le groupe comprenant des substances cationiques, anioniques, par exemple des acides gras et des glycols, par exemple le butylène glycol, le propylène glycol, le polyéthylène glycol, des polyglucosides, des stérols végétaux, des alcools stéariques, le behenet 25, le cétéaryl alcool.

Par « conservateur », on entend tout composé permettant d'inhiber le développement de microorganismes dans une composition cosmétique et/ou dermatologique. Ces composés sont connus de l'homme du métier. Il peut s'agir par exemple d'un agent fongicide et/ou d'un agent antibactérien.

Par « agent fongicide », on entend tout composé capable de diminuer et/ou d'inhiber la prolifération des champignons dans une composition cosmétique et/ou dermatologique.

Par « agent antibactérien », on entend tout composé capable de diminuer ou d'inhiber la prolifération de bactéries dans une composition cosmétique et/ou dermatologique. Par exemple, le conservateur peut être choisi dans le groupe comprenant les parabènes, par exemple le butylparabène, l'éthylparabène, le propylparabène, l'isobutylparabène, le méthylparabène, les acides organiques, les diazolidiines, les isothiazolinones, les hydroxyméthylglycinates, les alcools phénoliques, le 5-bromo-5-nitro-1,3 dioxane, diazolidinyl urée, le 3'-Déméthoxy-3O-Déméthylmatairésinol (DMDM) hydantoïne.

Par exemple, le silicone peut-être choisi dans le groupe comprenant les diméthicones, les diméticolols, les phényl triméthicones, les huiles de silicones volatiles et non volatiles.

Par « agent chélatant », on entend tout composé formant des complexes avec des ions métalliques modifiant la stabilité et/ou la viscosité d'une composition cosmétique et/ou dermatologique. Ces composés sont connus de l'homme du métier. Par exemple, l'agent chélatant peut être choisi dans le groupe comprenant l'acide nitroloacétique, l'acide polyaminocarboxylique, l'acide éthylènediaminetétraacétique (EDTA) et des sels de ceux-ci.

Par « agent épaississant », on entend tout composé capable d'augmenter la viscosité d'une composition cosmétique et/ou dermatologique. Ces composés sont connus de l'homme du métier. Par exemple, l'agent épaississant peut être choisi dans le groupe comprenant des polymères naturels comme l'acide alginique et ses dérivés, la cellulose et ses dérivés, les scléroglucanes, la gomme de xanthane, la gomme arabique, des polymères et copolymères carboxyvinyliques synthétiques qui peuvent avoir également une fonction d'émulsifiant par exemple l'acrylate, le méthacrylate, l'acrylamide et les mélanges de ceux-ci, par exemple le copolymère acrylate/acrylate d'alkyle en C10 à 30, le polyméthylméthacrylate.

Par « agent humectant », on entend tout composé capable de maintenir et/ou de conserver l'humidité d'une composition cosmétique et/ou dermatologique. Ces composés sont connus de l'homme du métier. Par exemple l'agent humectant peut être choisi parmi le butylène glycol, le propylène glycol, le polyéthylène glycol.

Par « antioxydant », on entend tout composé capable d'inhiber les réactions d'oxydation par l'oxygène et le rancissement. Ces composés sont connus de l'homme du métier. Par exemple, l'antioxydant peut être choisi dans le groupe comprenant les sulfites, les acides aminés, par exemple la glycine, l'histidine, la tyrosine, le tryptophane et des dérivés de ceux-ci, les imidazoles, des peptides, par exemple la D-carnosine, la L-carnosine.

Par « tensioactif », on entend un composé permettant de réduire la tension de surface de la composition et de favoriser une répartition uniforme des composés d'une composition cosmétique et/ou dermatologique. Ces composés sont connus de l'homme du métier. Par exemple, le tensioactif peut être choisi dans le groupe comprenant le cétyl bétaine, le cocamidopropyl bétaine, le disodium de cocoamphodiacétate, le disodium laureth sulfosuccinate, l'hexylèneglycol, le laureth-3, le laureth-4, le polyéthylène glycol (PEG) PEG-30, le glycéryl cocoate, le PEG-40 l'huile de castor hydrogénée, le PEG-7, le glycéryl cocoate, le polysorbate 20, le polysorbate 80, le cétyl phosphate de potassium, le cétéaryl sulfate de sodium, le laureth sulfate chimique de sodium, le laurylsulfate de sodium, le palmitate de sodium (hexadécanoate de sodium), le stéareth-100, le stéareth-2, le stéareth-21.

Par « agent absorbant », on entend un composé qui absorbe les substances hydrosolubles et/ou liposolubles dissoutes ou dispersées. Ces composés sont connus de l'homme du métier. Par exemple, l'agent absorbant peut être choisi dans le groupe comprenant le nitrure de bore, l'acide silicique, le carbonate de magnésium, la cellulose microcristalline, la silice (dioxyde de silicium), le talc (E553).

Par « agent filmogène », on entend un composé qui permet la formation d'un film continu lors de l'application d'une composition cosmétique et/ou dermatologique. Ces composés sont connus de l'homme du métier. Par exemple, l'agent filmogène peut être choisi dans le groupe comprenant le copolymère acrylamide/ammonium acrylate, le copolymère d'acrylamides, le copolymère de chlorure d'acrylamido-propyl-trimonium/acrylates, le copolymère acrylates acrylates/ alkyl C10-30 acrylate, la cire d'abeilles blanche (E901), le cyamopsis tétragonolubus (Guar), l'hydroxyéthylcellulose, le polyéthylène téréphtalate (PET), le polyglycérylméthacrylate, le polyquaternium-10, le polyquaternium-11, le polyquaternium-7, le copolymère polyvinylpyrrolidone (PVP)/Eicosène, le copolymère PVP/Hexadécène, le copolymère PVP/ vinylacétate (VA).

Par « agent stabilisateur », on entend un composé qui permet d'améliorer la stabilité d'une composition cosmétique et/ou dermatologique et/ou permet d'améliorer la stabilité des composants inclus dans ladite composition. Ces composés sont connus de l'homme du métier. Par exemple, l'agent stabilisateur peut être choisi dans le groupe comprenant l'hydroxyéthylcellulose, la cellulose microcristalline, la polycaprolactone, le stannate de sodium.

Par « agent tampon », on entend un composé qui permet de stabiliser le pH d'une composition cosmétique et/ou dermatologique. Ces composés sont connus de l'homme du métier. Par exemple, l'agent tampon peut être choisi dans le groupe comprenant l'aminométhyle propanol, l'acide citrique, l'acide fumarique, l'acide orthophosphorique, l'acide sébacique (acide décanedioïque), le citrate de sodium, l'hydroxyde de sodium, l'acide tartrique, le pyrophosphate de tétrasodium, la triéthylamine (TEA).

Par « filtre UV », on entend un composé permettant de filtrer certains rayons UV. Ces composés sont connus de l'homme du métier. Par exemple, le filtre UV peut être choisi dans le groupe comprenant la benzophénone-3 (oxybenzone), la benzophénone-4 (sulisobenzone), l'avobenzone, l'éthylhexylméthoxycinnamate, l'octylméthoxycinnamate, l'éthylhexyl salicylate

Par « hydratant », on entend un composé permettant d'augmenter la teneur en eau d'une composition cosmétique et/ou dermatologique. Ces composés sont connus de l'homme du métier. Par exemple, l'hydratant peut être choisi dans le groupe comprenant l'aloès (aloe vera), l'extrait végétal d'ananas, le diméthiconol, l'échinacea angustifolia, la glycine, l'extrait de réglisse, l'extrait de jasmin, les esters de jojoba, le stéarate de magnésium, le mannitol, le miel, l'huile de Ricin.

Par « liant », on entend un composé permettant d'augmenter la cohésion d'une composition cosmétique. Ces composés sont connus de l'homme du métier. Par exemple, le liant peut être choisi dans le groupe comprenant les copolymères d'acrylates, le cyamopsis tétragonolubus (Guar), l'hydroxyéthylcellulose, le myristate d'isopropyle, le carbonate de magnésium, le mannitol, le polyéthylène glycol-90M, le polydécène, le copolymère PVP/Eicosène, le copolymère PVP/Hexadécène, le copolymère PVPNA, le copolymère d'acrylates de sodium, le copolymère carboxyméthyl bétaglucan de sodium, le dextran carboxyméthyl de sodium, la cire de paraffine, la gomme de xanthane.

Par « stabilisateur d'émulsion », on entend un composé permettant d'améliorer l'émulsification d'une composition cosmétique et/ou dermatologique et/ou d'améliorer la stabilité d'une émulsion cosmétique et/ou dermatologique. Ces composés sont connus de l'homme du métier. Par exemple, le stabilisateur d'émulsion peut être choisi dans le groupe comprenant le stéarate acétylé de glycol, le carbomer, le cétéaryl d'alcool, le cyamopsis tétragonolubus, l'hydroxyéthylcellulose, la cellulose microcristalline, le PEG-90M, le copolymère PVPNA, le dextran carboxyméthyle de sodium, l'acide stéarique, l'octadécanol, la cire de synthèse de paraffine synthétique, la gomme de xanthane.

Par « parfum », on entend un composé permettant de donner à une composition cosmétique et/ou dermatologique une odeur particulière Ces composés sont connus de l'homme du métier. Par exemple, le parfum peut être tout parfum approprié pour une application cosmétique et/ou dermatologique. Le parfum peut être choisi par exemple dans le groupe comprenant les extraits naturels de plantes, les essences de plantes, par exemple des extraits et/ou essences de roses, de camomille, de menthe.

Le ou les composé(s) utilisé(s) pour constituer le produit cosmétique et/ou dermatologique dépend notamment de la formulation choisie pour l'utilisation cosmétique et/ou dermatologique de la composition de la présente invention.

La composition cosmétique et/ou dermatologique peut être sous toute forme appropriée pour son utilisation. Il peut s'agir par exemple d'une solution, d'une suspension, d'une émulsion, d'une microémulsion, d'une pâte. Il peut s'agir par exemple d'un gel, d'une crème, d'un aérosol, d'un onguent, d'une mousse, d'une poudre, d'une huile, de comprimés, de suppositoires, gélules, capsules, etc., éventuellement au moyen de forme galénique ou dispositif assurant une libération prolongée et/ou retardée. Pour ce type de formulation, on utilise avantageusement un agent tel que la cellulose, des carbonates ou de l'amidon.

La composition cosmétique et/ou dermatologique de l'invention peut également comprendre de l'eau. Dans ce cas, préférentiellement, la composition comprend de l'eau déminéralisée ou distillée.

Ladite composition peut également comprendre au moins un autre ingrédient actif, particulièrement un autre ingrédient thérapeutiquement et/ou cosmétologiquement et/ou dermatologiquement actif, par exemple pour une utilisation simultanée, séparée ou étalée dans le temps suivant la formulation galénique utilisée.

La composition peut comprendre en outre un moyen permettant de faciliter sa pénétration dans la peau. Par exemple un composé choisi dans le groupe comprenant les vasodilatateurs, les glucocorticoïdes Cela présente un intérêt lorsque la composition de la présente invention est destinée à une application topique.

La composition de la présente invention peut être utilisée par tout moyen approprié connu de l'homme du métier. Par exemple, en application topique, transdermique ou transcutanée, ou une composition administrable par voie orale. Elle peut également être utilisée dans un patch transdermique. Elle peut également être appliquée sur la peau au moyen d'un pulvérisateur ou par trempage.

Pour l'application de la composition, on peut utiliser également un moyen physique augmentant la pénétration de la composition dans la peau, par exemple un film plastique déposé ou enveloppant la peau après application de la composition.

Selon l'invention, dans la composition cosmétique et/ou dermatologique de l'invention, le rapport de l'inhibiteur de la farnésyl-pyrophosphate synthase sur l'inhibiteur d'hydroxyméthyl glutaryl coenzyme A réductase, ou de l'un de leurs sels physiologiquement acceptables, peut être compris entre 0,002 et 50, de préférence entre 0,005 et 25, de préférence entre 0,01 et 1,8, de préférence de 0,05 à 0,35.

Dans la composition cosmétique et/ou dermatologique de l'invention la quantité d'inhibiteur de la farnésyl-pyrophosphate synthase peut être comprise entre 0,001 à 0,500 pour cent en poids, de préférence de 0,005 à 0,025, de préférence de 0,025 à 0,1, de préférence de 0,100 à 0,150, de préférence de 0,150 à 0,200, de préférence de 0,200 à 0,250, de préférence de 0,250 à 0,300, de préférence de 0,300 à 0,350, de préférence de 0,350 à 0,400, de préférence de 0,400 à 0,450, de préférence de 0,450 à 0,500 pour cent en poids de la composition cosmétique et/ou dermatologique.

Dans la composition cosmétique et/ou dermatologique de l'invention la quantité d'inhibiteurs d'hydroxyméthyl glutaryl coenzyme A réductase peut être comprise entre 0,010 à 0,500 pour cent en poids, de préférence de 0,075 à 0,095, de préférence de 0,095 à 0,100, de préférence de 0,100 à 0,150, de préférence de 0,150 à 0,200, de préférence de 0,200 à 0,250, de préférence de 0,250 à 0,300, de préférence de 0,300 à 0,350, de préférence de 0,350 à 0,400, de préférence de 0,400 à 0,450, de préférence de 0,450 à 0,500 pour cent en poids de la composition cosmétique et/ou dermatologique.

Dans la composition cosmétique et/ou dermatologique de l'invention la somme de la quantité d'inhibiteurs d'hydroxyméthyl glutaryl coenzyme A réductase et de la quantité d'inhibiteur de la farnésyl-pyrophosphate synthase peut être comprise entre 0,010 et 1,000, de préférence de 0,1 à 0,800 pour cent en poids de la composition, de préférence la quantité est égale à 0,1, pour cent en poids.

D'autres avantages pourront encore apparaître à l'homme du métier à la lecture des exemples ci-dessous, donnés à titre illustratif et illustrés par les figures annexées.

### Brève description des figures

La Figure 1 illustre les résultats obtenus en Western Blot sur des fibroblastes témoins "normaux" traités avec des doses croissantes d'une statine hydrosoluble (pravastatine P, 20 à 100 µM), et d'un aminobiphosphonate (NBP, zolédronate Z, 20 à 100 µM) (Pistes A à I, respectivement P20/Z20, P20/Z60, P20/Z100, P60/Z20, P60/Z60, P60/Z100, P100/Z20, P100/Z60, P100/Z100). La piste J est un contrôle positif de présence de prélamine A (fibroblastes de patients DR), la piste K est le contrôle négatif, traité avec le solvant seul (PBS).

La Figure 2 illustre les résultats obtenus aux doses efficaces de chacun des produits.

La Figure 3 illustre l'effet supérieur obtenu lors de l'administration des 2 produits ensemble.

La Figure 4 illustre l'action de l'association des 2 produits sur des cellules âgées.

La Figure 5 illustre la multiplication cellulaire des fibroblastes par mesure de l'index mitotique en fonction des conditions de culture cellulaire. L'index mitotique correspond au rapport entre le nombre de noyaux marqués (entrant en division) par rapport au nombre de noyaux totaux du champ observé en fonction de chaque traitement.

Figure 6 : Western blot montrant que le blocage de la prénylation de la prélamine A nécessite à la fois l'inhibition de la farnésyl-transférase et de la géranyl-géranyl-transférase de type I. Détection de la lamine A/C dans des cellules HeLa traités par inhibiteurs de la farnésyl-transférase et/ou de la géranylgénarnyl transférase de type I. LA = lamine A, LC = lamine C, Pre = prélamine A.

Figure 7: La lamine A (cellules témoins) et la prélamine A (cellules de souris *Zmpste24*^{*-*/*-*}) ont été analysées par spectrométrie de masse (MALDI-ToF). **a, b** : portions du spectre correspondant aux peptides tryptiques farnésylés (**a**) et géranylgéranylés (**b**).

Figure 8: Analyse par spectrométrie de masse de protéines extraites de l'enveloppe nucléaire de cellules non traitées (**a**), de cellules de patients progéria traitées par FTI (2,5 µM, **b**) ou traitées par le mélange pravastatine+zolédronate (1 µM chacun, **c**).

Figure 9: Analyse par spectrométrie de masse de protéines extraites de l'enveloppe nucléaire de fibroblastes non traitées (**a**) ou traitées par le mélange pravastatine+zolédronate (1 µM chacun, **b**) provenant de souris *Zmpste24*^{*-*/}*⁻.*

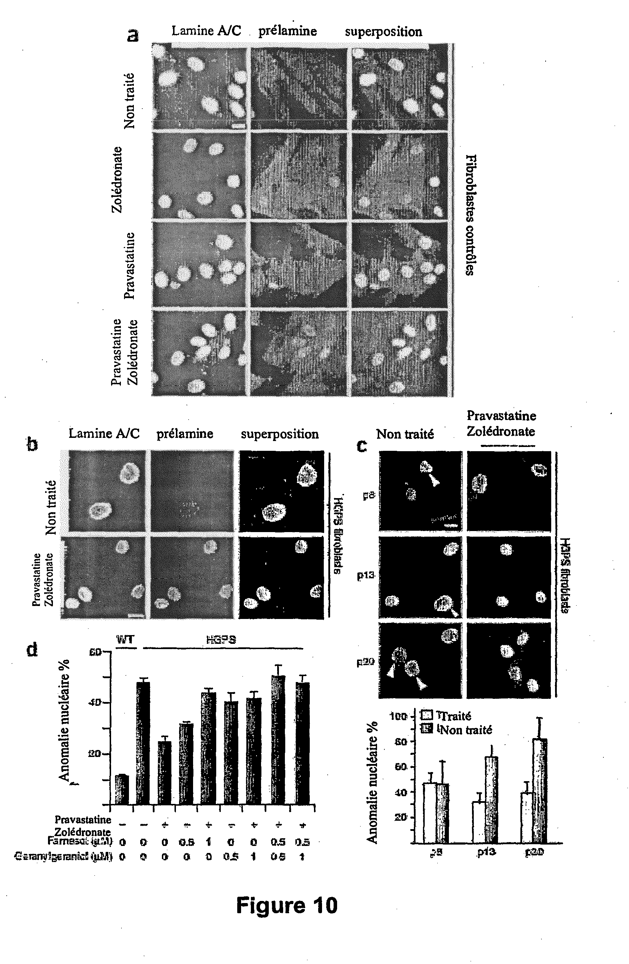

Figure 10: représentations de résultats de différentes expérimentations démontrant l'effet synergique de la combinaison pravastatine+zolédronate sur l'accumulation de prélamine A dans des cellules témoins et de patients progéria : (a) Détection immunocytochimique de la lamine A/C et de la prélamine A dans des fibroblastes humains normaux, non traités, traités par pravastatine et/ou par zolédronate. (**b**) Détection immunocytochimique de la lamine A/C et de la prélamine A dans des fibroblastes humains normaux et de patients progéria traités par la combinaison pravastatine+zolédronate. (**c**) Analyse quantitative de l'effet du traitement pravastatine+zolédronate sur la morphologie nucléaire de cellules de patients progéria. (**d**) Analyse quantitative de l'effet du traitement pravastatine+zolédronate sur la morphologie nucléaire de cellules de patients progéria en présence de farnésol, de géranylgéraniol ou des deux composés. Barres d'erreur = moyenne ± erreur standard de la moyenne. Barre d'échelle = 10 µm.

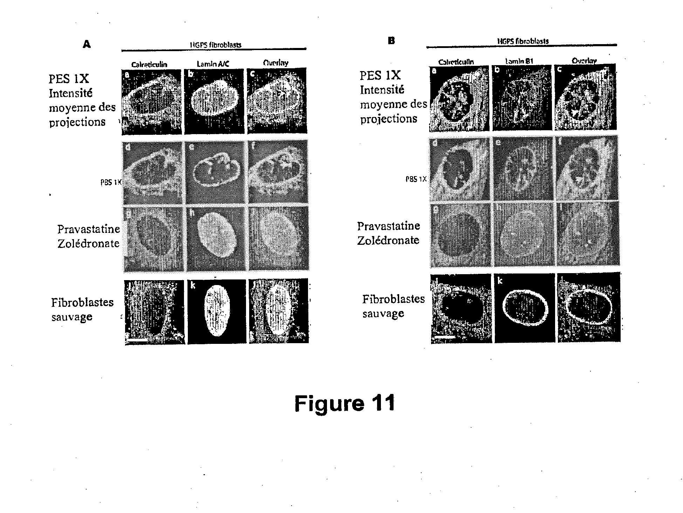

Figure 11 : Représentation de résultats de traitement par pravastatine+zolédronate montrant la correction de la morphologie nucléaire et l'induction d'une relocalisation partielle des isoformes de la lamine A/C et de la lamine B1 de la lamina nucléaire dans le nucléoplasme, dans des cellules de patients progéria. (**A**) Immunofluorescence et microscopie confocale. Les images **a** à **c** de chaque panneau sont des projections de l'intensité moyenne de 27 images de la pile et montrent les tubules du réticulum nucléaire marqués par la calréticuline dans les cellules progéria incubées avec le PBS. Images **d** à **l** : coupes confocales isolées de 0,2 µm d'épaisseur. Effet du traitement pravastatine+zolédronate **(g)** et **(h). (B)** Colocalisation de la lamine B1 et de la calréticuline. Barre d'échelle = 5 µm.

Figure 12 : Représentation de l'effet de la pravastatine et du zolédronate associés ou non sur la morphologie nucléaire de cellules de souris *Zmpste24*^{*-*/*-*} **(a)** et de souris témoins **(b)** en culture, en présence de farnésol, de géranylgéraniol ou des deux molécules.

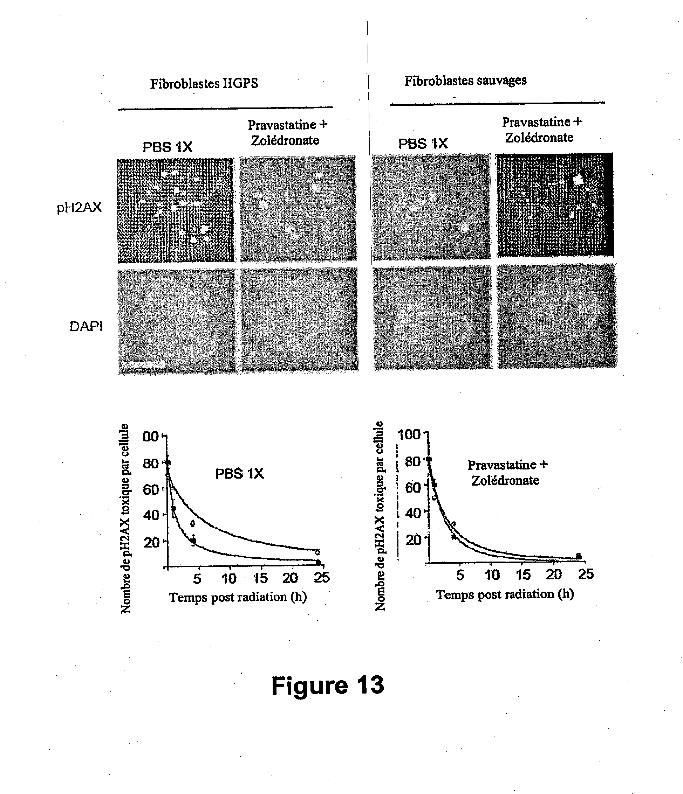

Figure 13: Effet du traitement pravastatine+zolédronate sur les anomalies de la réparation des cassures double-brin (DSB) de l'ADN dans les cellules de patients progéria. Immunodétection des foci de l'histone H2AX phosphorylée détectés 24 h après irradiation, foci correspondant aux cassures double brin non réparées (images du haut). Marquage nucléaire du DAPI (images du bas). Courbes du bas : évolution du nombre de foci d'histone H2AX phosphorylée en fonction du temps après irradiation dans les cellules témoins (carré plein) et les cellules progéria (cercle vide) incubées avec le PBS ou traités avec pravastatine+zolédronate. Chaque courbe représente la moyenne ± erreur standard de la moyenne d'au moins 3 expériences.

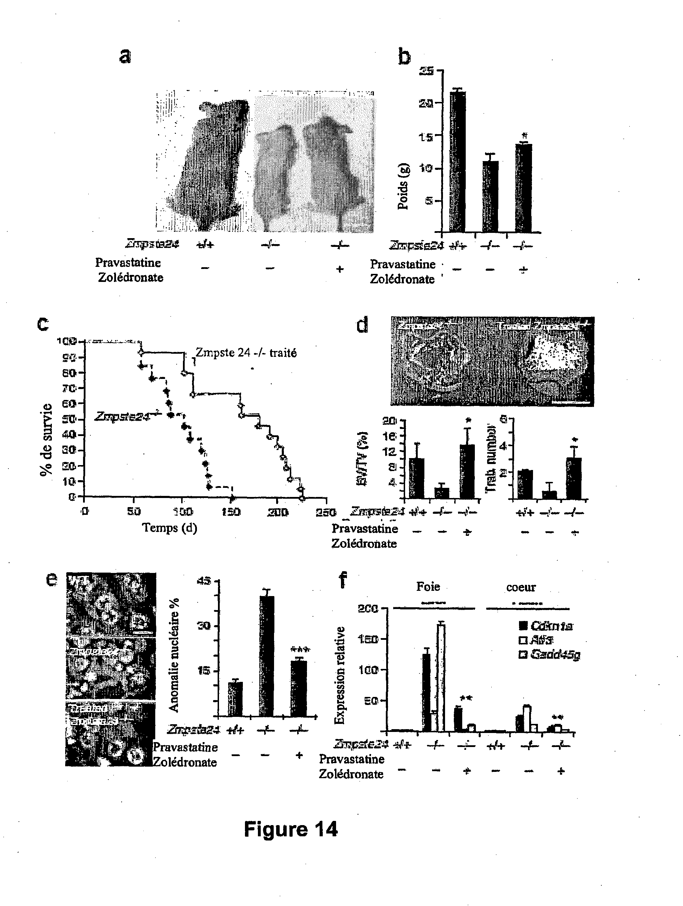

Figure 14: Effet du traitement pravastatine+zolédronate sur le phénotype progéroïde de souris *Zmpste24*^{*-*/*-*} : **(a)** Photographies représentatives de souris âgées de 3 mois *Zmpste24*^{+/+}*, Zmpste24*^{-/-} et *Zmpste2*4^{-/-} traitées par la combinaison pravastatine + zolédronate. Barre d'échelle = 1 cm. **(b)** Poids de souris âgées de 3 mois *Zmpste24*^{+/+} {n = 12), *Zmpste24*^{-/-} (n = 13) et *Zmpste24*^{-/-} traitées (n = 15). **(c)** Courbes de Kaplan-Meier montrant une augmentation significative de la dure de vie des souris *Zmpste24*^{-/-} traitées. **(d)** Représentation tridimensionnelle par microtomographie informatisée du tibia de souris *Zmpste24*^{-/-} traitée et non traitée (image du haut). Le panneau du bas représente le volume osseux relatif et le nombre de trabécules osseux dans les souris *Zmpste24*^{-/-} non traitées et traitées. **(e)** Quantification des anomalies nucléaires des hépatocytes de souris *Zmpste24*^{+/+}*, Zmpste24*^{-/-} et *Zmpste24*^{-/-} traitées. Les flèches blanches montrent les noyaux anormaux. Barre d'échelle = 10 µm. **(f)** Expression relative des gènes cibles de la p53 dans le foie et le coeur de souris *Zmpste24*^{+/+}, *Zmpste24*^{-/-} et *Zmpste24*^{-/-} traitées, analysée par RT-PCR quantitative. * P < 0,05 ; ** P < 0,01 ; *** P < 0,001. Les barres d'erreur représentent la moyenne ± erreur standard de la moyenne.

Figure 15: Effet de la pravastatine seule ou du zolédronate sur la durée de vie des souris Zmpste24^{+/+} : Courbes de Kaplan-Meier pravastine seule **(a)** et zolédronate seul **(b)** sur des souris *Zmpste24*^{-/-}, traitées (diamant vide) et non traitées (cercles pleins).

Figure 16: Effet d'un traitement pravastatine+zolédronate sur la durée de vie de souris *Lmna*^{-/-} : Courbes de Kaplan-Meier à partir de souris *Lmna*^{-/-} traitées par pravastine+zolédronate (n= 12, diamant vide), comparée à celle de souris non traitées (cercles pleins, n = 11).

### EXEMPLES

### EXEMPLE 1 : Effet synergique de l'association d'un inhibiteur de l'hydroxyméthylglutaryl-coenzyme A (HMG-CoA) réductase hydrosoluble (une statine hydrosoluble : la pravastatine) et d'un inhibiteur de la farnésyl-pyrophosphate synthase (un aminobiphosphonate : le zolédronate) sur des cultures de cellules normales et progéroïdes

### A. PROTOCOLES

### A.1 Cellules et culture cellulaire

Les lignées de cellules sont soit des fibroblastes témoins AG16409 provenant du Coriell Institute, soit des fibroblastes issus de biopsies de patients atteints de Dermopathie Restrictive. Elles sont cultivées à 37°C sous 5% de CO₂ en salle P2.

Le milieu de culture complet usuel est
- RPMI (Invitrogen) complémenté avec
- Sérum de Veau Foetal 20% (Invitrogen)
- L-Glutamine 200 mM (Invitrogen)
- Mélange Pénicilline/Streptomycine/Fungizone 1 X (Stock 100 X, Cambrex)

### A.2 Récolte des cellules

La récolte des cellules est réalisée par trypsinisation de la façon suivante (protocole pour une grande flasque, 75 cm², BD Falcon) :
▪ Le milieu est aspiré ;
▪ Les cellules sont lavées avec 10 ml de PBS 1X (Invitrogen) ;
▪ 5 ml d'une solution de Trypsine/EDTA 1X (Cambrex) sont ajoutés ;
▪ La flasque est incubée environ 6 min à 37°C, le temps que les cellules se décollent ;
▪ La trypsine est inhibée par dilution dans 15 ml de milieu complet ;
▪ Les cellules sont culottées par centrifugation 10 min à 1000 tours par minutes à 16°C ;
▪ Le culot est resuspendu dans 2 ml de PBS 1X, et re-centrifugé dans les mêmes conditions.

Les cellules obtenues sont soit congelées pour une utilisation ultérieure, soit repiquées à partir de ce culot lavé.

### A.3 Traitements

La solution de pravastatine (statine hydrosoluble) utilisée est préparée comme suit :

40 mg de pravastatine (Sigma Aldrich, P4498) sont repris dans de l'eau stérile pour constituer une solution stock à 10 mM.

Les concentrations finales testées ont été de 500 nM, 1, 5, 50 et 100 µM, obtenues en diluant la solution stock dans du milieu complet.

La solution de zolédronate (NBP) utilisée est préparée comme suit :

Une solution stock d'acide (1-hydroxy-2-imidazol-1-yl-1-phosphono-éthyl) phosphonique (0,8 mg/ml, Novartis) est ajustée à une concentration de 2 mM.

Les concentrations finales testées ont été de 500 nM, 1, 5, 50 et 100 µM, obtenues en diluant la solution stock dans du milieu complet.

### A.4 Western blot

### A.4.1 Préparation des cellules

Pour une expérience de Western blot, les cellules sont traitées comme suit :

Environ 7,5 x 10⁵ cellules sont ensemencées dans une grande flasque et cultivées dans les conditions ci-dessus jusqu'à presque confluence (4 jours).

Au bout de 4 jours, les cellules sont lavées avec du PBS 1X, et reprises dans du milieu complet supplémenté avec le traitement.

Les cellules sont incubées le temps du traitement (de 6 à 72h, séquentiellement ou simultanément) dans l'incubateur à 37°C.

À l'issue du traitement, les cellules sont trypsinisées (protocole ci-dessus) et le culot obtenu stocké à -80 °C jusqu'à extraction des protéines.

### A.4.2 Extraction des protéines pour Western blot

Le culot cellulaire est repris dans 300 µl de tampon de lyse

| | |
|---|---|
| Triton X100 | 1 % |
| SDS | 0,1 % |
| Désoxycholate de sodium | 0,5 % |
| NaCl | 50 mM |
| EDTA | 1 mM |
| TrisHCl pH 7,4 | 20 mM |
| Inhibiteur de Protéase (Roche 11697498001) | 1 pastille pour 50 ml |

Extemporanément, on ajoute

| | |
|---|---|
| Orthovanadate de sodium | 1 mM |
| PMSF | 1 mM |

Les cellules sont exposées à une sonication 2 fois 30 sec *(Brandson Sonifier Cell Disruptor B15*).

Les débris cellulaires sont centrifugés pendant 10 min à 10 000 tours par minutes à 4 °C.

Le surnageant protéique est conservé à -80 °C jusqu'à utilisation.

Le dosage des protéines est effectué à la décongélation.

### A.4.3 Western blot

### - Gel

Un gel d'acrylamide 8 % est classiquement utilisé pour détecter les différentes formes de lamines A/C.

| | | |
|---|---|---|
| Acrylamide/bisacrylamide 37/1 | 8 | % |
| TrisHCl pH 8,8 | 375 | mM |
| SDS | 0,1 | % |
| APS | 0,1 | % |
| TEMED | 0,01 | % |

Un gel de concentration est coulé sur le gel séparatif

| | | |
|---|---|---|
| Acrylamide/bisacrylamide 37,5/1 | 3 | % |
| TrisHCl pH 6.8 | 375 | mM |
| SDS | 0,1 | % |
| APS | 0,1 | % |

| | | |
|---|---|---|
| TEMED | 0,01 | % |

La concentration en protéines des échantillons est dosée, et des aliquots sont ajustés à 50 µg par tube dans du tampon de lyse en qsp 15 µl.

5 µl de tampon de charge sont ajoutés à chaque échantillon

| | | |
|---|---|---|
| SDS | 4 | % |
| TrisHCl pH 6,8 | 100 | mM |
| Glycérol | 20 | % |
| β-mercaptoéthanol | 20 | % |
| Bleu de bromophénol | traces | |

Les échantillons sont dénaturés par chauffage 5 min à 95 °C et déposés dans les puits.

La migration a lieu à 50, puis 100 Volts, dans un tampon :

| | | |
|---|---|---|
| Tris-Base | 0,3 | % |
| Glycine | 1,44 | % |
| SDS | 0,1 | % |

### - Transfert

La membrane de transfert (Hybon P, Amersham Biosciences) est préparée par trempage dans l'éthanol, suivi d'un bain de 5 min dans l'eau stérile, et de 10 min dans le tampon de transfert :

| | | |
|---|---|---|
| Tris-Base | 12 | mM |
| Glycine | 96 | mM |
| Ethanol | 20 | % |

Le gel est humidifié pendant 20 min dans le tampon de transfert, puis le sandwich est monté (système Miniprotéan, Biorad).

Le transfert a lieu en général sur la nuit, en chambre froide, à 10 Volts.

La membrane est rincée dans du PBS 1X, conservée à l'humidité, et utilisé telle quelle pour la détection.

### - Détection

La membrane est incubée 1h à température ambiante dans une solution de saturation :

| | |
|---|---|
| Caséine | 10 % |
| Tween 20 | 0,1 % |
| PBS | 1 X |

On la rince 2 fois 10 min dans du tampon de lavage (Tween 20 0,1% / PBS 1X).

L'anticorps primaire est dilué dans le tampon de saturation (détails et dilution, voir ci-dessous immunomarquage).

La membrane est incubée avec les anticorps primaires 1h à température ambiante sous agitation.

A l'issue, elle est rincée 3x avec du tampon de lavage, puis lavée 3x 15 min avec le même tampon.

L'anticorps secondaire (système couplé à la péroxydase, Jackson Immunoresearch) est dilué au 1/10000^{e} dans du tampon de saturation..

La membrane est incubée avec cette solution 30 à 45 min à température ambiante sous agitation.

A l'issue, elle est rincée 3x avec du tampon de lavage, puis lavée 3x 15 min avec le même tampon.

La détection est effectuée avec le kit *ECL Plus Western Blotting System* d'Amersham Bioscience, selon les indications du fournisseur.

Après révélation, la membrane est exposée sur film *Biomax MR* (Kodak), le temps nécessaire pour avoir un signal satisfaisant.

### A.5 Immunomarquage

### A.5.1 Préparation des cellules

Une culture de cellules est trypsinisée, et les cellules comptées sur cellule de Neubauer.

Des puits de culture style Labtech (Nunc, réf. 177399) sont ensemencés, à raison de 5x10⁴ cellules par puits.

Le milieu de culture complet est supplémenté par le ou les traitements (statine, NBP, les 2), et les cellules cultivées pendant le temps *ad hoc.*

A l'issue, le milieu de culture est aspiré, les puits démontés.

Les lames sont lavées dans du PBS 1X.

Les cellules sont fixées dans une solution de paraformaldéhyde 4% (dans PBS) pendant 10 minutes à température ambiante.

Un lavage de 10 min dans du PBS 1X est effectué.

Les cellules sont déshydratées par des bains successifs de 3 min dans des solutions de pourcentage éthanolique croissant (70, 90, 100%, ce dernier bain étant répété).

Après séchage, les lames sont stockées à -80°C jusqu'à utilisation.

### A.5.2 Marquage

Après décongélation, les cellules sont incubées 5 min à température ambiante en chambre humide dans 50 µl d'une solution de perméabilisation :

| | |
|---|---|
| PBS | 1 X |
| Triton X100 | 0,5 % |
| RNS (Rabbit Normal Sérum, Vector S5000) | 5 % |
| Inhibiteur de Protéase (Roche 11697498001) | 1 pastille pour 50 ml |

3 bains de préincubation de 15 min chacun sont effectués dans 50 µl de la solution d'incubation :

| | |
|---|---|
| PBS | 1 X |
| RNS | 5 % |
| Inhibiteur de Protéase (Roche 11697498001) | 1 pastille pour 50 ml |

L'anticorps primaire est dilué au 1/100^{e} dans 50 µl de solution d'incubation et mis au contact des cellules pendant 1 h à température ambiante en chambre humide.

Les anticorps primaires utilisés sont de 2 types :
- Souris anti-lamine A/C (côté N-terminal), clone 4A7, don de G. Morris (Oswestry, UK)
- Chèvre anti-prélamine A (15 aa extrémité C-terminale), produit SC6214, Santa Cruz Biotechnology, Inc.

3 rinçages dans 50 µl de PBS 1X sont effectués pendant 15 min chacun.

L'incubation avec l'anticorps secondaire a lieu pendant 1 h dans 50 ml de solution d'incubation à température ambiante en chambre humide. Les anticorps secondaires sont de deux types :
- Ane anti-souris, Jackson Immunoresearch, dilution au 1/100^{e}
- Ane anti-chèvre, Jackson Immunoresearch, dilution au 1/200^{e}

3 rinçages dans 50 µl de PBS 1X sont effectués pendant 15 min chacun.

Une incubation avec 100 µl de solution DAPI 50 ng/ml (SERVA, réf. 18860) est réalisée pendant 15 min à température ambiante en chambre humide.

3 rinçages dans du PBS 1X sont effectués dans des bacs porte-lames pendant 5 min chacun.

Un ultime rinçage est effectué pendant 5 min dans une solution de Tween20 à 0,1% dans du PBS.

### A.5.3 Montage

Les cellules sont immergées dans une goutte de VectaShield (Vector), recouvertes d'une lamelle couvre-objet et observées sur un microscope à fluorescence (Leica DMR, Leica Microsystems), équipé d'un système de caméra coolSNAP (Princeton).

### B. RESULTATS

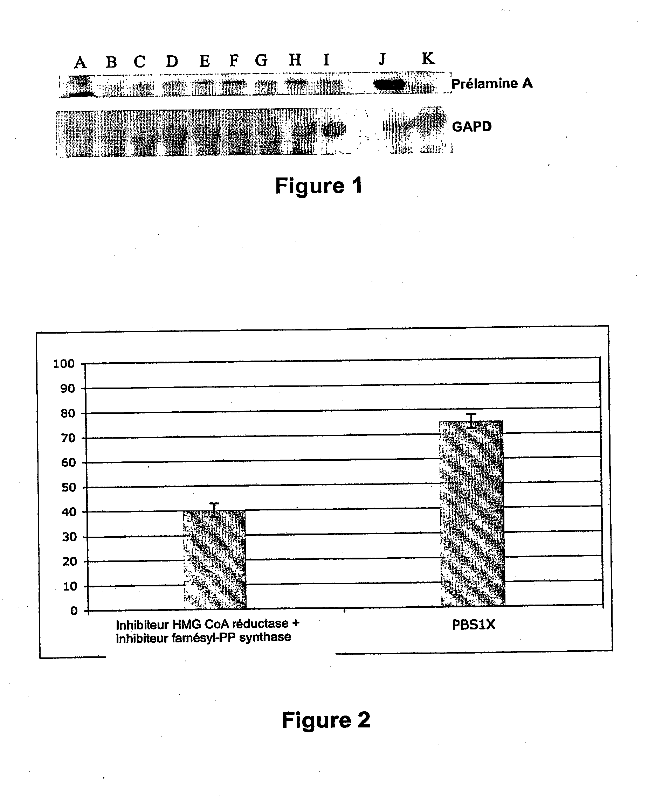

### B.1. Western blot (Figure 1)

Des fibroblastes témoins "normaux" ont été traités avec une statine hydrosoluble (pravastatine P, 20 à 100 µM), et avec un aminobiphosphonate (NBP zolédronate Z, 20 à 100 µM) en association (Pistes A à I, respectivement P20/Z20, P20/Z60, P20/Z100, P60/Z20, P60/Z60, P60/Z100, P100/Z20, P100/Z60, P100/Z100). Le western-blot montre "l'apparition" d'une bande correspondant à la taille de la prélamine A non mature (non tronquée) en fonction de l'augmentation de concentration des deux molécules, ce qui confirme que la farnésylation est nécessaire à la maturation de la lamine A. Ce résultat montre que le blocage de la synthèse du farnésyl-PP en 2 points de la voie métabolique est plus efficace qu'un blocage en un seul point sur l'inhibition de la farnésylation de la prélamine A, au moins *ex-vivo.*

### B.2. Dose- et durée-réponse en immunohistochimie (Figure 2)

Des courbes dose-réponse et durée-réponse ont permis de déterminer une efficacité maximale en mesurant 2 paramètres sur des cellules témoins saines d'une part, puis sur des cellules de patients HGPS.

L'association pravastatine (hydrosoluble) / zolédronate (NBP) la plus efficace a été obtenue pour une administration de 1 µM de pravastatine pendant 24 h, de zolédronate pendant 12 h sur les cellules saines. Aucune toxicité n'a été observée. Sur les cellules HGPS (Cellules avec anomalies nucléaires), en utilisant le même protocole d'administration, le nombre de noyaux "déformés" a baissé de 75 à 40 %. La mesure du taux de prélamine A obtenu sur cellules saines a été effectuée. Cette mesure a montré que ce taux est maximal.

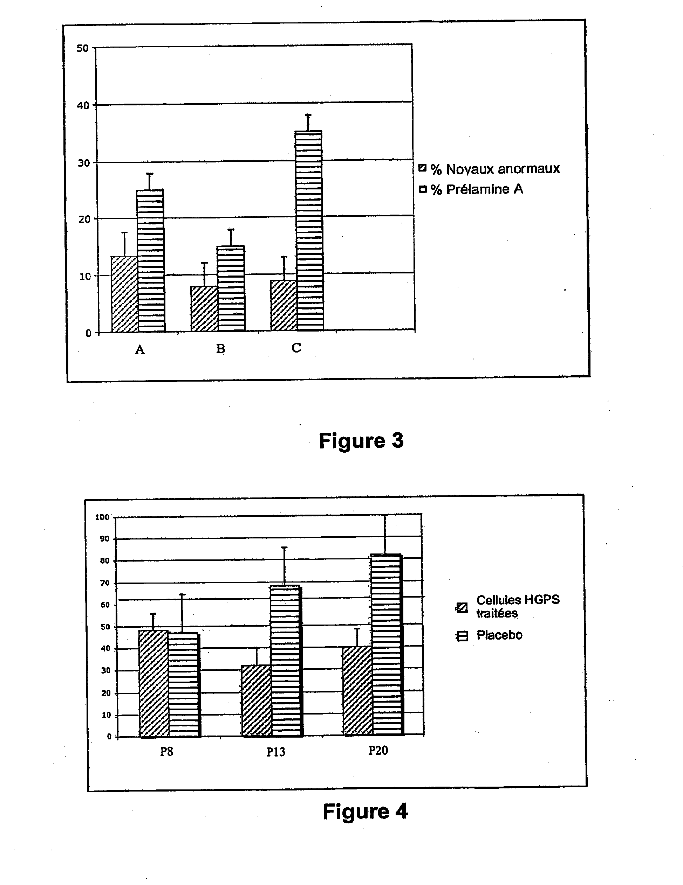

### B.3. Effet du traitement en immunohistochimie (Figure 3)

L'action combinée de la pravastatine et du zolédronate, traitement: Pravastatine 100 µM pendant 12h, Zolédronate 20 µM pendant 6h, montre une meilleure efficacité, puisque le taux de prélamine A produit dans des cellules saines traitées (estimé à 35%) est beaucoup plus élevé en association que si les molécules sont ajoutées seules (respectivement 25 et 15 %). De plus, le taux de noyaux déformés (signe d'une toxicité sur les cellules saines) est minimal (inférieur à 10%), et inférieur à ce qu'il est sur des cellules traitées avec de la pravastatine seule (environ 12 %).

### B.4. Action sur des cellules âgées en immunohistochimie (Figure 4)

En fonction du nombre de "passages" (nombre de repiquage des cellules), donc de l'âge des cellules, la proportion de noyaux anormaux augmente. Cette caractéristique est typique des cellules HGPS non traitées. Si on traite les cellules HGPS avec la Pravastatine 1 µM pendant 24h et Zolédronate 1 µM pendant 12h., cette proportion se maintient, et diminue même un peu (moins de 40% contre plus de 80% chez les cellules traitées avec un placébo).

### B.5. Conclusion

L'association pravastatine/zolédronate est efficace à des doses pour lesquelles quasiment aucun effet n'est observé avec les molécules administrées séparément.

L'effet physiologique de blocage de la voie de prénylation est donc obtenu avec des doses bien inférieures à celles utilisées en traitement unique dans des articles publiés sur des cultures de cellules (Kusuyama & al 2006 **(27),** 10 µM de pravastatine seule sur progéniteurs de cellules vasculaires ; Flint & al 1997 **(13),** 25 µM de pravastatine seule sur cellules de muscle de rat néonatal).

### EXEMPLE 2 : Effet d'une composition cosmétique selon l'invention comprenant un inhibiteur de l'hydroxyméthylglutaryl-coenzyme A (HMG-CoA) réductase hydrosoluble et d'un inhibiteur de la farnésyl-pyrophosphate synthase sur la division de fibroblaste humains âgés et sur des fibroblastes humains jeunes

### A. OBJET DE L'EXEMPLE

Dans le présent exemple, l'évaluation de l'effet *in vitro* d'une composition selon l'invention comprenant un inhibiteur de l'hydroxyméthylglutaryl-coenzyme A (HMG-CoA) réductase hydrosoluble ou liposoluble et d'un inhibiteur de la farnésyl-pyrophosphate synthase sur le taux de division cellulaire (index mitotique) de fibroblastes a été mesuré. Une comparaison de l'effet de la composition sur des fibroblastes humains âgés par rapport à des fibroblastes humains jeunes a également été effectuée. Le nombre d'actifs utilisés dans cette expérience est de quatre, les produits ayant été employés en combinaisons deux par deux. Les actifs utilisés sont:
A1 : Zolendronate
A2 : Alendronate
B1 : Pravastatine
B2 : Simvastatine

Les associations particulières qui ont été utilisées dans cet exemple sont : A1B1, A1B2, A2B1, A2B2.

### B. PROTOCOLE

Dans cet exemple, deux lots de fibroblastes, fibroblastes âgés (n° de lot : 9052) et jeunes (n° de lot : 7080), ont été mis en culture dans un milieu RPMI (Invitrogen) contenant 10 % de sérum de veau foetal sans antibiotiques durant 24 h après trypsination de boîtes fournies.

Les différents actifs ont été ajoutés à une concentration de 1 µM finale chacun durant 24 h (une dilution 1000 d'une solution stock dans l'eau pour les composés A1, A2 et B1, ou dans l'Ethanol 100% pour le composé B2, a été effectuée).

L'index mitotique a été évalué par incorporation de Bromo-déoxy-uridine (BrdU) sur 45 minutes après 24 h d'incubation des cellules avec l'une des combinaisons d'actifs. Une révélation immunohistochimique a permis de révéler les cellules en phase de synthèse d'ADN (cellule en pré-division). Une coloration des noyaux (du matériel génétique) a été réalisée par incorporation de di-amino phényl indol (DAPI).

La saisie de 6 champs microscopiques (OLYMPUS IX 70) a permis une mesure de l'index mitotique par analyse d'image (OLYMPUS AnalySIS). L'index mitotique correspond au rapport du nombre de noyaux ayant incorporé le BrdU sur le nombre de noyaux ayant incorporé le DAPI. Un index moyen est calculé statistiquement avec une déviation standard (écart-type) comprise entre 0,005 et 0,061.

Un test de Student bilatéral a permis de mesurer la signification statistique des résultats obtenus.

### C. RESULTATS

### C.1. Observation visuelle générale des cellules

Ces résultats montrent une très faible capacité de division des fibroblastes âgés en l'absence de tout traitement, préalablement à l'étude.

Les fibroblastes jeunes ont montré une capacité de division supérieure à celle des fibroblastes âgés. La capacité de division des fibroblastes âgés était inférieure à 5 %, tandis que la capacité de division des fibroblastes jeunes était de 15,6 %. La différence de capacité de division entre les fibroblastes âgés non traités et les fibroblastes jeunes non traités était donc égale à 3.

Au cours de la réalisation de cet exemple, aucune toxicité n'a été observée visuellement après 24h d'incubation des fibroblastes avec les combinaisons d'actifs testées.

Au cours de la réalisation de cet exemple, aucun effet toxique de l'éthanol (0.1 % final) n'a été observé après 24h d'incubation.

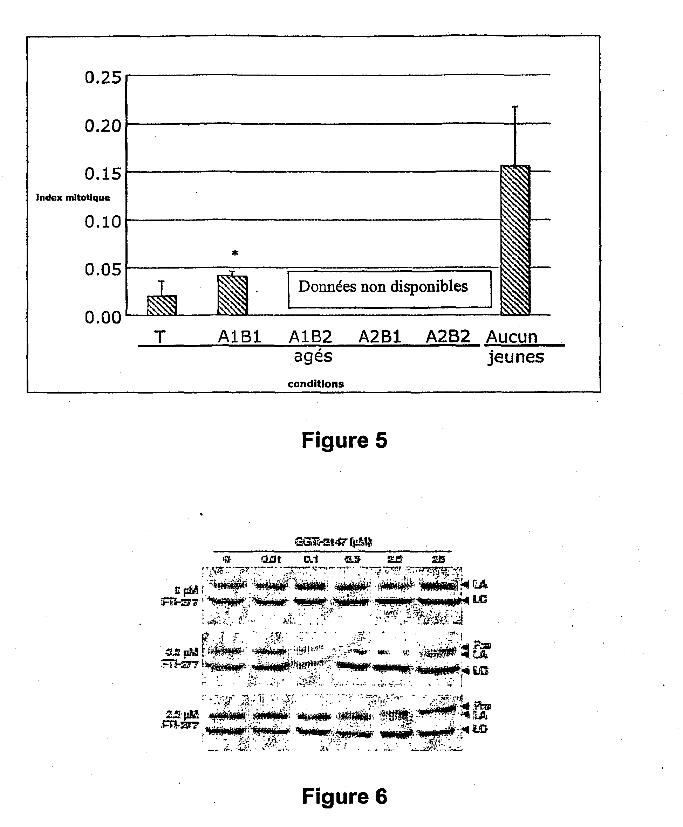

### D. EVALUATION DE L'INDEX MITOTIQUES (Figure 5)

D'une manière générale, le nombre de fibroblastes âgés sans aucun traitement en phase de synthèse d'ADN était extrêmement faible : moins de 5% (voir figure 5, colonne 1).

L'index mitotique n'était pas non plus très élevé pour les fibroblastes jeunes : de l'ordre de 15 % (voir figure 5, colonne 6).

Par comparaison avec les fibroblastes âgés témoins sans aucun traitement, les fibroblastes témoins exposés à l'éthanol (0.1% - 24 h), ne présentent pas une différence significative (p = 0.11, n=6) de leur index mitotique. Les valeurs ont été alors regroupées (Témoin, n=12).

Les résultats présentés dans la figure 5, colonne 2, montrent, sur l'index mitotique des fibroblastes âgés un effet activateur de A1B1 = Zolendronate-Pravastatine par rapport au témoin (stimulation d'un facteur 2 maximum) (p < 0.001, n≥6).

Cet exemple montre donc que l'application de la combinaison Zolendronate-Pravastatine a un effet activateur de la division cellulaire de fibroblastes de sujet âgé.

### EXEMPLE 3: Effet de l'association d'un inhibiteur de l'hydroxyméthylglutaryl-coenzyme A (HMG-CoA) réductase hydrosoluble et d'un inhibiteur de la farnésyl-pyrophosphate synthase sur un modèle de souris présentant un syndrome progéroïde

Les souris KO *Zmpste24*^{-/-} utilisées ici sont celles décrites dans l'article cité de Varela & al. 2005 **(49).** Une preuve d'efficacité de l'association des 2 molécules (pravastatine et zolédronate) a été apportée en collaboration avec un laboratoire espagnol (Pr C. Lopez-Otin). L'efficacité est obtenue à des doses combinées qui n'ont pas d'effet lorsque les produits sont utilisés séparément, démontrant un effet synergique.

Les 2 molécules (Acide zolédronique (Zometa (marque déposée)) 100 µg/kg/jour et Pravastatine 100 mg/kg/jour) ont été diluées dans du PBS 1X et injectées par voie intrapéritonéale, quotidiennement, sur des souris âgées de 1 mois et jusqu'à leur décès. Les contrôles sont des souris sauvages de même portée, traitées au PBS 1X seul.

La survie des souris traitées a été grandement améliorée, elle est maximale notamment pour les femelles, avec un allongement de la durée de vie moyenne de 80% environ. Les symptômes cliniques de la maladie sont tous considérablement réduits par rapport aux individus traités avec le PBS seul. Notamment, il a été observé un effet du traitement sur la peau et la repousse des poils de ces souris par rapport aux souris traitées avec le PBS, qui montraient de larges zones glabres.

### EXEMPLE 4 : compositions cosmétiques selon l'invention comprenant un inhibiteur de l'hydroxyméthylglutaryl-coenzyme A (HMG-CoA) réductase hydrosoluble et un inhibiteur de la farnésyl-pyrophosphate synthase

**Composition 1:**

| Ingrédients | Pourcentage |
|---|---|
| Pravastatine | 0.075 |
| Acide Alendronique | 0.025 |
| Alcool Dénaturé. | 15.000 |
| PEG-8 Diméthicone | 5.000 |
| Glycéryl Polyméthacrylate | 4.950 |
| Glycérine | 3.000 |
| PEG-400 | 3.000 |
| Butylène Glycol | 1.070 |
| Phénoxyéthanol | 0.430 |
| Parfum (Fragrance) | 0.350 |
| Copolymer Acrylates/C10-30 Alkyl Acrylate | 0.320 |
| PPG-26-Buteth-26 | 0.220 |
| Chlorphenesin | 0.200 |
| Trométhamine | 0.170 |
| PEG-40 huile de castor Hydrogénée | 0.140 |
| Dioxyde de titane, Mica, Silice | 0.300 |
| Méthylparabène | 0.120 |
| Propylène Glycol | 0.050 |
| Laureth-3 | 0.050 |
| Ethylparabène | 0.024 |
| Butylparabène | 0.024 |
| Hydroxyéthylcellulose | 0.020 |
| Propylparabène | 0.015 |
| Sodium Hyaluronate | 0.005 |
| Eau | QS 100 |

**Composition 2 :**

| Ingrédients | Pourcentage |
|---|---|
| Pravastatine | 0.075 |
| Acide Alendronique | 0.025 |
| Glycérine | 5.000 |
| Beheneth-25 | 5.000 |
| Dicaprylyl Carbonate | 4.000 |
| Octyldodécanol | 3.000 |
| Cétéaryle d'alcool | 3.000 |
| Glycéryle Polyméthacrylate | 2.970 |
| Butylène Glycol | 2.010 |
| Diméthicone | 2.000 |
| Décyl Oléate | 2.000 |
| Huile d'amande douce | 2.000 |
| Cétyl Palmitate | 2.000 |
| Polyméthylméthacrylate | 1.000 |
| Phénoxyéthanol | 0.430 |
| Butylparabène | 0.424 |
| Sodium Polyacrylate | 0.300 |
| Copolymère Acrylates/C10-30 Alkyl Acrylate | 0.300 |
| Chlorphenesine | 0.300 |
| Tocophéryl Acétate | 0.250 |
| Parfum (Fragrance) | 0.200 |
| Laureth-3 | 0.150 |
| Méthylparabène | 0.120 |
| Disodium EDTA | 0.100 |
| Gum de Xanthane | 0.100 |
| Hydroxyde de Sodium | 0.089 |
| Hydroxyéthylcellulose | 0.060 |
| PEG-8 (polyéthylène glycol) | 0.049 |
| Propylène Glycol | 0.030 |
| Tocophérol | 0.025 |
| Ethylparabène | 0.024 |
| Propylparabène | 0.015 |
| Isobutylparabène | 0.010 |
| Ascorbyl Palmitate | 0.004 |
| Acide Ascorbique | 0.0008 |
| Acide Citrique | 0.0008 |
| Eau | QSP 100 |

**Composition 3 :**

| Ingrédients | Pourcentage |
|---|---|
| Caprylique/Caprique Triglycéride | 5.000 |
| Glycéryl Polyméthacrylate | 4.950 |
| Stéareth-21 | 3.000 |
| Glycérine | 3.000 |
| Polyméthyl Méthacrylate | 3.000 |
| Butylène Glycol | 2.010 |
| Stéareth-2 | 2.000 |
| Cyclopentasiloxane | 1.505 |
| Butyrospermum Parkii ( beurre de karité) | 1.000 |
| Huile d'amande douce | 1.000 |
| Laureth-3 | 0.750 |
| Sodium PCA | 0.523 |
| Phénoxyéthanole | 0.430 |
| Carbomer | 0.300 |
| Tocophéryl Acétate | 0.250 |
| Boron Nitride | 0.250 |
| Chlorphénesine | 0.250 |
| Copolymère de Diméthicone | 0.245 |
| Parfum (Fragrance) | 0.200 |
| Polyacrylamide | 0.197 |
| Méthylparabène | 0.120 |
| C13-14 Isoparaffine | 0.100 |
| Disodium EDTA | 0.100 |
| Hydroxyde de Sodium | 0.0875 |
| Hydroxyéthylcellulose | 0.060 |
| Propylène Glycole | 0.050 |
| Laureth-7 | 0.025 |
| PEG-8 (Polyéthylène glycol), Tocophérol, Ascorbyle Palmitate, Acide Ascorbique, Acide Citrique | 0.080 |
| Ethylparabène | 0.024 |
| Butylparabène | 0.024 |
| Propylparabène | 0.015 |
| C.I 42090 (Blue 1) | 0.0002 |
| Pravastatine | 0.075 |
| Acide Alendronique | 0.025 |
| Eau | QS 100 |

**Composition 4 :**

| Ingrédients | Pourcentage |
|---|---|
| Caprylic/Capric Triglycéride | 5.000 |
| Glycéryl Polyméthacrylate | 4.950 |
| Stéareth-21 | 3.000 |
| Glycérine | 3.000 |
| Polyméthyl Méthacrylate | 3.000 |
| Butylène Glycol | 2.010 |
| Stéareth-2 | 2.000 |
| Cyclopentasiloxane | 1.505 |
| Butyrospermum Parkii (beurre de karité) | 1.000 |
| Huile d'amande douce | 1.000 |
| Laureth-3 | 0.750 |
| Sodium PCA | 0.523 |
| Phénoxyéthanol | 0.430 |
| Carbomer | 0.300 |
| Tocophéryl Acétate | 0.250 |
| Boron Nitride | 0.250 |
| Chlorphénesine | 0.250 |
| Copolymère de Diméthicone | 0.245 |
| Parfum (Fragrance) | 0.200 |
| Polyacrylamide | 0.197 |
| Méthylparabène | 0.120 |
| C13-14 Isoparaffine | 0.100 |
| Disodium EDTA | 0.100 |
| Hydroxyde de Sodium | 0.0875 |
| Hydroxyéthylcellulose | 0.060 |
| Propylène Glycol | 0.050 |
| Laureth-7 | 0.025 |
| PEG-8, Tocophérol, Ascorbyl Palmitate, Acide Ascorbique, Acide Citrique | 0.080 |
| Ethylparabène | 0.024 |
| Butylparabène | 0.024 |
| Propylparabène | 0.015 |
| C.I 42090 (Blue 1) | 0.0002 |
| Pravastatine | 0.095 |
| Acide zolendronique | 0.005 |
| Eau | QS 100 |

**Composition 5 :**

| Ingrédients | Pourcentage |
|---|---|
| Alcool Dénaturé. | 15.000 |
| PEG-8 Diméthicone | 5.000 |
| Glycéryl Polyméthacrylate | 4.950 |
| Glycérine | 3.000 |
| PEG-400 | 3.000 |
| Butylène Glycol | 1.070 |
| Phénoxyéthanol | 0.430 |
| Parfum (Fragrance) | 0.350 |
| Copolymère Acrylates/C10-30 Alkyl Acrylate | 0.320 |
| PPG-26-Buteth-26 | 0.220 |
| Chlorphénesine | 0.200 |
| Trométhamine | 0.170 |
| PEG-40 huile de castor hydrogénée | 0.140 |
| Dioxide de titane, Mica, Silice | 0.300 |
| Méthylparabène | 0.120 |
| Propylène Glycol | 0.050 |
| Laureth-3 | 0.050 |
| Ethylparabène | 0.024 |
| Butylparabène | 0.024 |
| Hydroxyéthylcellulose | 0.020 |
| Propylparabène | 0.015 |
| Sodium Hyaluronate | 0.005 |
| Pravastatine | 0.095 |
| Acide zolendronique | 0.005 |
| Eau | QSP 100 |

**Composition 6 :**

| Ingrédients | Pourcentage |
|---|---|
| Glycérine | 5.000 |
| Beheneth-25 | 5.000 |
| Dicaprylyl Carbonate | 4.000 |
| Octyldodécanol | 3.000 |
| Cétéaryl Alcool | 3.000 |
| Glycéryl Polyméthacrylate | 2.970 |
| Butylène Glycol | 2.010 |
| Diméthicone | 2.000 |
| Décyl Oléate | 2.000 |
| Huile d'amande douce | 2.000 |
| Cétyl Palmitate | 2.000 |
| Polyméthylméthacrylate | 1.000 |
| Phénoxyéthanol | 0.430 |
| Butylparabène | 0.424 |
| Polyacrylate de Sodium | 0.300 |
| Acrylates/C10-30 Alkyl Acrylate Copolymère | 0.300 |
| Chlorphénésine | 0.300 |
| Tocophéryl Acétate | 0.250 |
| Parfum (Fragrance) | 0.200 |
| Laureth-3 | 0.150 |
| Méthylparabène | 0.120 |
| Disodium EDTA | 0.100 |
| Gomme de Xanthane | 0.100 |
| Hydroxyde de Sodium | 0.089 |
| Hydroxyéthylcellulose | 0.060 |
| PEG-8 (Polyéthylène Glycol) | 0.049 |
| Propylène Glycol | 0.030 |
| Tocophérol | 0.025 |
| Ethylparabène | 0.024 |
| Propylparabène | 0.015 |
| Isobutylparabène | 0.010 |
| Ascorbyle Palmitate | 0.004 |
| Acide Ascorbique | 0.0008 |
| Acide Citrique | 0.0008 |
| Pravastatine | 0.095 |
| Acide Zolendronique | 0.005 |
| Eau | QSP 100 |

**Composition 7 :**

| **Ingrédients** | **Pourcentage** |
|---|---|
| eau déminéralisée | 86,678% |
| Cetiol A (marque déposée) (hexyl Laurate) | 5,000% |
| Glycérine codex | 3,000% |
| Simulsol 165 (marque déposée) (stéarate de glycéryl) | 1,500% |
| Montanov 14 (marque de commerce) (alcool de Myristyl & glucoside myristyl) | 1,000% |
| Phénonip (marque déposée)(phénoxyéthanol, méthylparabène, butylparabène, éthylparabène, propylparabène) | 0,600% |
| Solution aqueuse NaOH à 10% | 0,512% |
| Covabsorb (marque de commerce) (éthylhéxyl Methoxycinnamate, Butyl méthoxydibenzoylméthane, éthylhexyl Salicylate, PPG-26-Buteth-26, hydrogéné Polyéthylèneglycol G-40, huile de Castor) | 0,500% |
| Pravastatine | 0,500% |
| Carbopol (marque enregistrée) (acide prop-2-enoic) | 0,300% |
| Chlorphénésine | 0,250% |
| Sepigel 305 (marque de commerce) (Polyacrylamide et Isoparaffine en C13-14 et Laureth-7) | 0,100% |
| Alendronate | 0,060% |
| | 100,000% |

**Composition 8 :**

| **Ingrédients** | **Pourcentage** |
|---|---|
| eau déminéralisée | 87,218% |
| Cetiol A (marque déposée) (hexyl Laurate) | 5,000% |
| Glycérine codex | 3,000% |
| Simulsol 165 (marque déposée) (stéarate de glycéryl) | 1,500% |
| Montanov 14 (marque de commerce) (alcool de Myristyl & glucoside myristyl) | 1,000% |
| Phénonip (marque déposée)(phénoxyéthanol, méthylparabène, butylparabène, éthylparabène, propylparabène) | 0,600% |
| Solution aqueuse NaOH à 10% | 0,512% |
| Covabsorb (marque de commerce) (éthylhéxyl Methoxycinnamate, Butyl méthoxydibenzoylméthane, éthylhexyl Salicylate, PPG-26-Buteth-26, hydrogéné Polyéthylèneglycol G-40, huile de Castor) | 0,500% |
| Carbopol (marque enregistrée) (acide prop-2-enoic) | 0,300% |
| Chlorphénésine | 0,250% |
| Sepigel 305 (marque de commerce) (Polyacrylamide et Isoparaffine en C13-14 et Laureth-7) | 0,100% |
| Pravastatine | 0,010% |
| Alendronate | 0,010% |
| | 100,000% |

### EXEMPLE 5 : Tests d'irritation cutanée sur des peaux reconstituées de compositions cosmétiques selon l'invention comprenant un inhibiteur de l'hydroxyméthylglutaryl-coenzyme A (HMG-CoA) réductase hydrosoluble et un inhibiteur de la farnésyl-pyrophosphate synthase

Dans le présent exemple, des compositions cosmétiques comprenant un inhibiteur de l'hydroxyméthylglutaryl-coenzyme A (HMG-CoA) réductase (une statine : la pravastatine) et un inhibiteur de la farnésyl-pyrophosphate synthase, l'acide zolendronique ou l'acide alendronique sont appliqués sur des épidermes humains reconstitués *in vitro.* La dimension des dermes est de 0,5 cm². Différentes quantités de la composition cosmétique sont appliquées, comprises entre 30 à 50 mg.

La viabilité cellulaire est déterminée par un test « MMT ». Le test « MTT » est un test colorimétrique basé sur la quantification des déshydrogénases mitochondriales, enzymes signant la viabilité cellulaire. Ce test est effectué à deux temps d'applications différents : 24 et 72 heures.

Des contrôles positifs utilisant du Sodium Dodécyl Sulfate (SDS) et des contrôles négatifs sont également effectués.

### EXEMPLE 6 : Tests d'irritations oculaire aiguë de compositions cosmétiques selon l'invention comprenant un inhibiteur de l`hydroxyméthylglutaryl-coenzyme A (HMG-CoA) réductase hydrosoluble et un inhibiteur de la farnésyl-pyrophosphate synthase

Dans le présent exemple, les tests sont réalisés selon les « bonnes pratiques de laboratoire » conformément aux exigences réglementaires, l'arrêté du 10/08/04 et l'article L5131-5 du code de la santé publique.

L'évaluation de la cytotoxicité est effectuée par la technique de relargage du rouge neutre sur des cellules de cornée de la lignée SIRC.

### EXEMPLE 7: Effets de l'association d'un inhibiteur de l'hydroxyméthylglutaryl-coenzyme A (HMG-CoA) réductase hydrosoluble et d'un inhibiteur de la farnésyl-pyrophosphate synthase sur des extraits ex-vivo de peau humaine.

### A. Protocole

Dans le présent exemple, les tests sont réalisés sur une peau provenant d'un donneur âgé d'environ 60 ans. Une préparation de 21 explants de peau humaine est réalisée et mise en survie en milieu BEM (BIO-EC's Explants Médium).

Les explants sont répartis en 3 lots de six explants et un lot témoin T0 de 3 explants, comme suit :
- **T0** Témoin Plastie : 3 explants
- **T** Témoin non traité : 6 explants
- **R** Contrôle positif : 6 explants
- **P** Explants traités par la composition de l'invention : 6 explants

### A.1. Traitement

Le traitement est réalisé à différent jour, au premier jour (J0), 2 heures après la préparation des explants, puis à J+1 jour, J+2 jours, J+4 jours, J+6 jours, J+8 jours et J10+ jours.

Les produits sont appliqués sur les explants comme suit :
- **T :** les explants ne reçoivent aucun traitement,
- **R:** les explants reçoivent chacun à J0, J+2 et J+4, 1 mg du contrôle positif (crème au rétinol)
- **P1** : les explants reçoivent chacun et à chaque temps de traitement 2mg du produit P,

Le traitement est réalisé par application topique de la composition de l'invention. La composition est ensuite répartie sur toute la surface de l'explant, à l'aide d'une spatule. La moitié du milieu de culture est renouvelée tous les deux jours et les explants sont remis en survie à 37 °C en atmosphère humide enrichie de 5% de CO₂.

### A.2. Prélèvement pour l'histologie

A J0, les 3 explants du lot T0 sont prélevés.

A J+6 jours et J+11 jours, 3 explants de chaque lot sont prélevés. Les prélèvements sont coupés en deux, une moitié est fixée dans du formol et l'autre moitié est congelée à -80°C, suivant la procédure BIO-EC « P006-PPEH ».

### B. Etude histologique

Après 24 heures de fixation dans le formol, les prélèvements sont déshydratés, imprégnés et enrobés en paraffine. Des coupes de 5µm sont réalisées pour l'observation morphologique.

### B.1. Première étape : étude morphologique

L'étude morphologique des structures épidermiques et dermiques est réalisée sur les coupes colorées au trichrome de Masson, variante de Goldner.

### B.2. Deuxième étape :

### B.2.1 Immunomarquage du KI67 :

L'immunomarquage des cellules en mitoses est réalisé sur coupes congelées avec l'anti-corps polyclonal anti-KI 67 (Novo Castra) révélé en DAB. Les cellules positives sont comptées sur toute la longueur épidermique et les moyennes ramenées au nombre de cellules marquées par cm.

### B.2.2 Immunomarquage du collagène I :

L'immunomarquage du collagène I sera réalisé sur coupes congelées avec l'anticorps polyclonal anti-collagène I révélé en FITC. Les noyaux sont contre-colorés à l'iodure de propidium.

### B.2.3 Immunomarquage du collagène III :

L'immunomarquage du collagène III est réalisé sur coupes congelées avec l'anti-corps polyclonal anti-collagène III révélé en DAB. Les noyaux sont contre-colorés à l'hémalun de Masson.

### B.2.4 Immunomarquage du collagène IV :

L'immunomarquage du collagène IV est réalisé sur coupes congelées avec l'anti-corps polyclonal anti-collagène IV (Cliniscience) révélé en FITC. Les noyaux sont contre colorés à l'iodure de propidium.

### B.2.5 Immunomarquage du collagène VII :

L'immunomarquage du collagène VII est réalisé sur coupes congelées avec l'anti-corps monoclonal anti-collagène VII révélé en FITC. Les noyaux sont contre- colorés à l'iodure de propidium.

### B.2.6 Immunomarquage de PECAM1 :

La visualisation des cellules endothéliales est réalisée grâce à l'immunomarquage de PECAM-1, réalisé sur coupes congelées avec l'anticorps monoclonal anti-PECAM-1 révélé en Fast-red.

### EXEMPLE 8 : Effet de l'association d'un inhibiteur de l'hydroxyméthylglutaryl-coenzyme A (HMG-CoA) réductase hydrosoluble et d'un inhibiteur de la farnésyl-pyrophosphate synthase sur des cultures in-vitro de cellules constitutives de la peau.

Cet exemple est réalisé avec les mêmes combinaisons d'actifs que dans l'exemple 2 ci-dessus. Ces différentes combinaisons d'actifs sont utilisées *in vitro* afin d'évaluer leur effet sur des paramètres physiologiques intervenant dans le vieillissement cutané.

Les combinaisons utilisées dans le présent exemple sont A1B1, A1B2, A2B1, A2B2, respectivement. Ces quatre combinaisons sont testées à plusieurs concentrations, les expériences étant faites en triple (ce qui représente au moins 36 points expérimentaux).

Les concentrations des 4 combinaisons sont proposées par le demandeur et il n'est donc pas envisagé à ce stade d'étude (sauf à titre de contrôle simple) de cytotoxicité *in vitro.* L'expérimentation est faite sur des cultures de fibroblastes tels que présentées dans l'exemple 1. Ce test est également appliqué à des cultures de kératinocytes. Les paramètres suivants sont examinés pour les 4 combinaisons d'actifs aux concentrations indiquées.
- Mesure de l'index mitotique.
- Mesure du remodelage de la matrice extracellulaire par contraction de lattices de collagène.
- Mesure de la réparation d'ADN génomique après irradiation aux UVB (Stress photoinduit s'approchant des conditions du bain de soleil)

La mesure de l'index mitotique est réalisée après exposition des cellules aux actifs durant un temps unique. L'index est évalué par comptage en analyse d'image des noyaux cellulaires ayant incorporé un analogue de la thymidine rendu fluorescent, sur le nombre de noyaux totaux. Plusieurs champs sont analysés. Des photos sont archivées pour iconographie.

Le remodelage de la matrice extracellulaire induite par les fibroblastes exposées aux actifs est évalué par incorporation de ces cellules dans des lattices de collagène et en quantifiant leur capacité à rétracter ces lattices. L'évaluation de la surface rétractée donne un index de remodelage. Des photos sont archivées pour iconographie.

La mesure de la réparation d'ADN génomique est réalisée après irradiation des cellules à une dose d'UV-B mimant les conditions d'un coup de soleil. Il est envisagé, dans un premier temps, d'évaluer l'effet des actifs au cours de la réparation d'ADN suivie sur une cinétique de 3 temps. La quantification est réalisée par détection et analyse d'image des dimères de pyrimidine cyclobutane induits par l'irradiation UVB à l'aide d'une technique immunohistochimique.

Des photos sont archivées pour iconographie.

### Exemple 9 : Un traitement associant une statine et un aminobiphosphonate augmente la durée de vie d'un modèle de souris reproduisant un syndrome humain de vieillissement prématuré.

Cet exemple est également publié dans Varela et al, Nature Medecine 2008, 7, 767 **(55).**

### Matériel et méthodes

### Souris :

La production des souris *Zmpsle24*^{-/-} *et Lmna*^{-/-} a été décrite (Pendas et al. 2002 **(38);** Sullivan et al., 1999 **(56)**)**.** La microtomographie informatisée osseuse des souris a été réalisée à l'aide du micro-CT SkyScan 1172 system (SkyScan - marque de commerce)). Toutes les expérimentations sur les souris obéissent aux règles édictées par le Comité de l'Expérimentation Animale de l'Université d'Oviédo (Espagne). La pravastatine (100 mg/kg/jour) et le zolédronate (100 mg/kg/jour) dilués dans du PBS sont administrés aux souris tous les jours. Les souris recevant le traitement pravastatine-zolédronate ou les souris témoins recevant uniquement du PBS ne présentent aucun dommage apparent ni stress.

### Culture cellulaire

Les fibroblastes dermiques d'un sujet témoin (GM00038) et de patients atteints de progéria et porteurs de la mutation G608G (AG11498 et AG01972) ont été obtenus du Coriell Cell Repository. Les cellules HeLa proviennent de l'American Type Culture Collection. Les cellules sont cultivées dans du DMEM (Gibco) supplementé avec 10% FBS (Gibco) et 1% antibiotic-antimycotic (Gibco). Les fibroblastes proviennent de queues de souris âgées de 12 jours (Varela et al., 2005). La concentration et la durée du traitement avec la statine et l'aminobiphosphonate sont indiquées dans la légende des figures. Lors du traitement combiné statine+aminobiphosphonate en présence de farnésol et/ou de géranylgéraniol, 1mM de pravastatine et 1mM de zolédronate ont été ajoutés au milieu de culture avec des concentrations croissantes de farnésol et/ou de géranylgéraniol. Le pourcentage de noyaux anormaux est mesuré 48 h après le début du traitement.

### Immunocytochimie

Les fibroblastes sont cultivés dans des chambres Lab Tek (Nalgen Nunc International), lavés dans le PBS, puis fixés dans du paraformaldéhyde 4% pendant 15 min. Les cellules sont déshydratées dans des bains d'éthanol en concentration croissante et sont perméabilisées 5 min à 25 °C dans du PBS contenant du Triton X-100 (0,5%), 5% de sérum

(chèvre ou lapin). Les lames sont préincubées à 25°C dans du PBS pendant 5 min.

La dilution des anticorps primaires est de 1:100 pour l'anticorps polyclonal de chèvre anti-prélamine A (Sc-6214, Santa Cruz Biotechnologies), 1:40 pour l'anticorps anti-lamine A/C (4A7 fourni par G. Morris), 1:200 pour l'anticorps de lapin anti-calréticuline (Stressgen) et 1:100 pour l'anticorps anti-lamine B1 (Abcam). Après lavage dans le PBS, les lames sont incubées 1 h à 25°C avec les anticorps secondaires dilués dans la solution d'incubation. La dilution des anticorps secondaires est la suivante : 1:100 pour l'IgG d'âne anti-souris couplé au FITC (Jackson ImmunoResearch), 1:400 pour l'IgG d'âne anti-chèvre couplé à l'Alexa 488, 1:800 pour l'IgG d'âne anti-chèvre couplé à l'Alexa 568 (Molecular Probes) et 1:100 pour l'IgG d'âne anti-lapin couplé au tétramethylrhodamine isothiocyanate (Sigma). Les cellules sont ensuite lavées, les noyaux colorés pendant 15 min à 25°C au DAPI (100 ng/ml, Sigma-Aldrich), enfin lavées 3 fois avec du PBS contenant 0,5% de Tween 20. Les lames sont montées dans du Vectashield (Vector). Des images numériques sont enregistrées avec un microscope Leica DMR équipé d'une caméra CoolSnap ou avec un microscope confocale Leica TCS SP5. Les noyaux sont observés dans des cellules après marquage de la lamine A/C. Plus de 100 noyaux ont été analysés dans des fibroblastes témoins pour chacun des traitements. Le nombre de noyaux de cellules de patients atteints de progéria analysé est de 250 (passage 8), 198 (passage 13) et 150 (passage 20).

### Irradiation X et étude de l'histone H2AX phosphorylée.

Les cellules de patients progéria et les cellules témoins 1 BR3 sont irradiées avec un appareillage X Philips. Le faisceau X est produit par une anode de tungstène soumise à une tension de 200 kV sous une intensité de 20 mA avec un filtre de cuivre de 0,1 mm de diamètre. Le débit de dose est de 1,243 Gy/min. L'histone phosphorylée H2AX est détectée avec des anticorps reconnaissant spécifiquement la sérine 139 phosphorylée (Upstate Biotechnology-Euromedex, Mundolsheim, France) à la dilution 1:800 et des anticorps anti-souris conjugués au FITC (1:100, Sigma-Aldrich). Le nombre des cassures double brin (DSB) en fonction de la durée de la réparation a été ajusté avec la formule Nt = NO (1/1 + βt)^{α}, où α et β sont des paramètres ajustables et Nt et NO le nombre de DSB au temps t et au temps 0.

### Western blot

Les cellules sont homogénéisées dans le milieu suivant : 50 mM Tris (pH 7,4), 150 mM NaCl, 1% NP-40, 50 mM NaF, 1 mM dithiothréitole, 2 mg/ml pepstatine A, en présence des inhibiteurs de protéases (Complete inhibitor cocktail, Roche) et d'inhibiteurs de phosphatases (Phosphatase Inhibitor Cocktails I and II, Sigma). Après électrophorèse, les protéines sont transférées sur des membranes de nitrocellulose bloquées avec 5% de poudre de lait délipidée, à l'aide du tampon TBS-T (20 mM Tris pH 7,4, 150 mM NaCl et 0,05% Tween-20), et incubées 1 h avec soit un anticorps anti-lamine A/C spécifique (4A7, 1:500), soit un anti-lamine A spécifique (C20, Santa Cruz Biotechnology, 1:250) ou un anti-bêta actine (A5441, Sigma, 1:5000). Les anticorps sont dilués dans du TBS-T contenant 3% de poudre de lait délipidée. Les blots sont ensuite incubés avec un anticorps couplé à la peroxydase (chèvre anti-souris ou anti-lapin, Jackson ImmunoResearch) dans du TBS-T contenant 1,5% de poudre de lait délipidée, puis lavés, enfin révélés par chémiluminescence (Immobilon Western chemiluminescent HRP substrate, Millipore - marque de commerce).

### Analyse par spectrométrie de masse

Les fibroblastes de souris témoins et *Zmpste24*^{-/-} ainsi que les cellules lymphoblastoïdes de patients progéria ont été homogénéisées, les noyaux isolés par ultracentrifugation et les protéines nucléaires obtenues comme décrit dans Blobel et Potter, V.R. Nuclei from rat liver : isolation method that combines purity with high yield, Science 154, 1662-1665, 1966. Les protéines de la lamina nucléaire ont été séparées par électrophorèse SDS-PAGE, et les bandes contenant la lamine A, la prélamine A et la progérine ont été excisées. Les fragments du gel ont été lavés deux fois avec 180 ml d'un mélange ammonium bicarbonate/acétonitrile (70:30, 25 mM), séchés (15 min, 90°C) et digérés (1 h, 60°C) avec de la trypsine (12 ng/ml, Promega) dans le bicarbonate d'ammonium 25 mM. Dans une expérimentation typique, 1 ml de CHCA (a-cyano-4-hydroxycinnamic acid, Waters) est placé dans un spectromètre MALDI-ToF. Une fois séché, 1 ml de la solution de peptides et 1 ml de la matrice (CHCA) sont placés dans le spectromètre équipé avec une source laser (Voyager-DE STR (marque de commerce), Applied Biosystems). Les données collectées à partie de 200 tirs laser produisent des spectres analysés avec le programme Data Explorer (version 4.0.0.0, Applied Biosystems).

### PCR quantitative en temps réel

Le taux d'expression de gènes cibles de p53 *(Atf3, Gadd45g* et *Cdkn1a,* qui code p21) a été mesuré avec l'appareil ABI PRISM 7900HT Sequence détection system (Applied Biosystems).

### Analyse statistique

La différence entre les différents groupes de souris et de cellules, traitées ou non, a été analysée à l'aide du teste de Student. Les calculs ont été réalisés à l'aide du programme Microsoft Excel. Les données sont exprimées en moyenne ± erreur standard de la moyenne (SEM).

### Résultats

Des cellules HeLa sont cultivées en présence d'inhibiteurs de la farnésyl-transférase (FTI-277, Sigma-Aldrich) et/ou de la géranyl-géranyl transférase de type I (GGTI-2147, Sigma-Aldrich) aux concentrations indiquées. Seule la combinaison des deux inhibiteurs conduit à l'accumulation dans les cellules d'une quantité importante de prélamine A non prénylée par rapport à l'effet de chacun des inhibiteurs utilisés seuls.

Ces résultats sont montrés sur la figure 6 qui est une photographie d'un Western blot obtenu montrant la Détection de la lamine A/C dans des cellules HeLa traités par inhibiteurs de la farnésyl-transférase et/ou de la géranylgénarnyl transférase de type I. LA = lamine A, LC = lamine C, Pre = prélamine A.

Ces résultats confirment que le blocage de la prénylation de la prélamine A nécessite à la fois l'inhibition de la farnésyl-transférase et de la géranyl-géranyl-transférase de type I conformément à la présente invention.

### L'inhibiteur de la farnésyl-transférase (FTI) induit la géranylgéranylation compensatoire de la progérine (dans des cellules de patients atteints de progéria) et dans des fibroblastes de souris Zmpste24^{-/-}

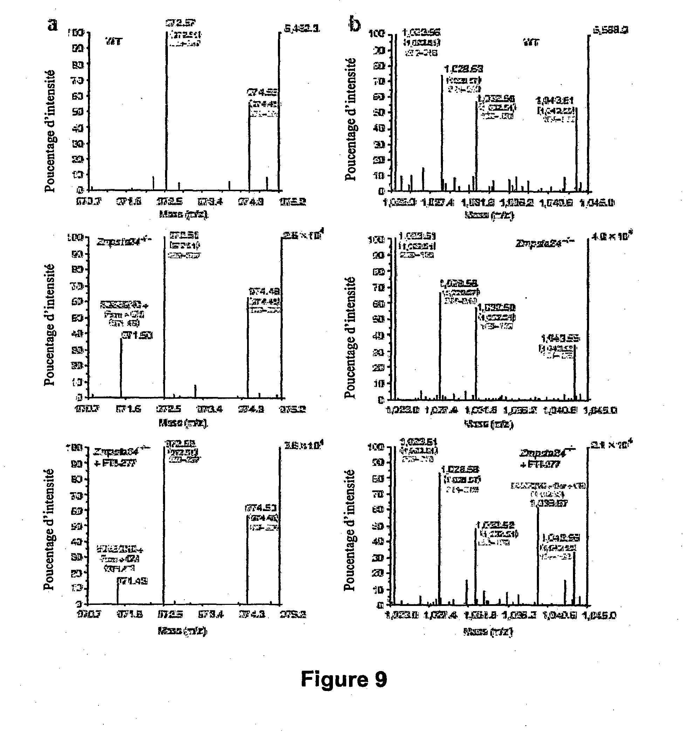

L'analyse par spectrométrie de masse montre comme attendu la présence de peptides tryptiques de la prélamine A farnésylée et carboxyméthylée dans les cellules de souris *Zmpste24*^{-/-} mais pas dans les cellules de souris témoins. Ces résultats sont montrés sur la Figure 9a. Le peptide farnésylé est dépourvu des 3 résidus SIM ce qui montre que Zmpste24 n'est pas indispensable au premier clivage au cours de la maturation de la prélamine A. Une diminution de la quantité de prélamine A farnésylée est observée dans les cellules de souris traitées par FTI. Lors de l'observation de la partie du spectre correspondant aux peptides géranylgéranylés, aucun peptide dérivé de la prélamine A n'a pu être détecté dans les cellules de souris *Zmpste24*^{*-*/*-*} non traitées. Mais un peptide dont la masse est compatible avec un fragment géranylgéranylée de la prélamine A est détecté après traitement avec le FTI . Ces résultats sont montrés sur la Figure 9b.

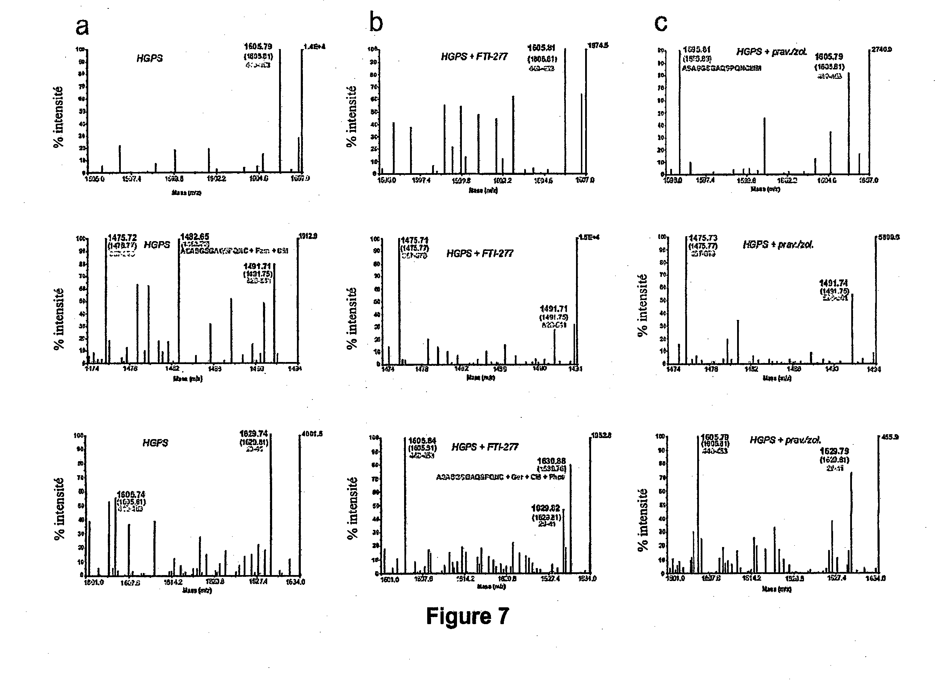

Dans les cellules de patients progéria, des peptides correspondant à la progérine farnésylée et carboxyméthylée sont détectés en l'absence de tout traitement, comme indiqué que la Figure 7a. Le traitement de ces cellules par FTI-127 provoque l'apparition de peptides dont la masse correspond à celle de peptides géranylgéranylés de la progérine, comme cela apparaît sur la Figure 7b.

L'ensemble de ces données montre que la progérine et la prélamine A sont géranylgéranylées de manière alternative sous l'effet des FTI et fournit une explication à la faible efficacité des traitements par FTI dans les modèles murins de syndrome progéroïdes.

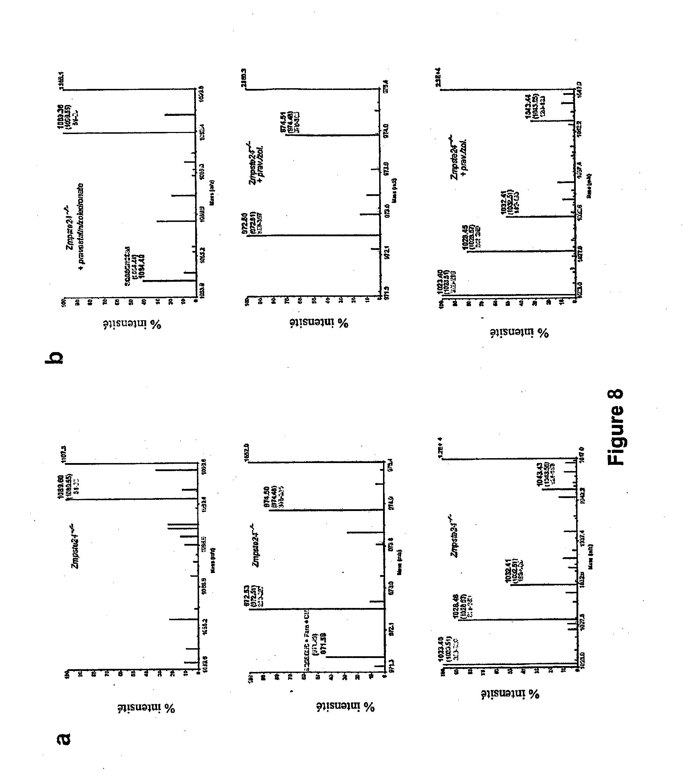

Les cellules de patients progéria et de souris *Zmpste24*^{*-*/*-*} ont été utilisées pour évaluer des stratégies thérapeutiques destinées à prévenir la prénylation croisée de la prélamine A et de la progérine. Nous avons fait l'hypothèse que la farnésylation des variants anormaux de la lamine A pourraient être inhibée par des drogues agissant sur la voie de biosynthèse du farnésyl-pyrophosphate, substrat de la farnésyl-transférase et précurseur du géranylgéranyl pyrophosphate, substrat de la géranyl-géranyl transférase de type 1. Nous avons donc testé l'effet de deux drogues, une statine et un aminobiphosphonate, connues pour agir à deux étapes de cette voie métabolique, sur des cellules de patients progeria. L'analyse par spectrométrie de masse montre que l'association pravastatine (statine) zolédronate (aminobiphosphonate) provoque l'apparition d'un peptide correspondant à l'extrémité C-terminale non prénylée de la progérine, peptide non détectable dans les cellules traitées par FTI, alors que ni les peptides farnésylés, ni les peptides géranylgéranylés ne sont pas non plus détectés, comme cela apparaît sur la Figure 7c). Le traitement statine+aminobiphosphonate inhibe bien la prénylation de la progérine. La même observation a été réalisée pour la prélamine A, comme cela apparaît sur la Figure 8. Son peptide C-terminal, non prénylé, est détecté dans les cellules traitées par le mélange statine+aminobiphosphonate, alors qu'il est absent des cellules non traitées, dans lesquelles on retrouve le peptide farnésylé et carboxyméthylé Enfin, le traitement pravastatine+zolédronate ne fait pas apparaître la prélamine A géranylgéranylée, à la différence du FTI.

Légende de la figure 9: La lamine A (cellules témoins) et la prélamine A (cellules de souris *Zmpste24*^{*-*/*-*}) ont été analysées par spectrométrie de masse (MALDI-ToF). **a, b** : portions du spectre correspondant aux peptide tryptiques farnésylés (a) et géranylgéranylés (b). Chacun des pics est marqué avec la masse théorique (entre parenthèses) du peptide provenant de la digestion par la trypsine de la lamine A ou de la prélamine A Le numéro des résidus est noté en bleu. La séquence des peptides et leur masse sont notées en rouge. Farn = farnesylé ; CM = carboxyméthylé ; Ger = géranylgéranylé.

Légende de la figure 7: Analyse par spectrométrie de masse de protéines extraites de l'enveloppe nucléaire de cellules non traitées (**a**), de cellules de patients progéria traitées par FTI (2,5 µM, **b**) ou traitées par le mélange pravastatine+zolédronate (1 µM chacun, **c**). La portion des spectres correspondant aux protéines non modifiées, farnésylées et géranylgéranylées est montrée en haut, au milieu et en bas de la figure. Chaque pic correspond au peptide tryptique de la progérine et est marqué avec la masse monoisotopique mesurée dans l'expérience et par la masse théorique (entre parenthèses). Le numéro des résidus d'acides aminés est noté en bleu. La séquence des peptides et leur masse sont notées en rouge.

La progérine est majoritairement farnésylée (F) et carboxyméthylée (Cm) dans les cellules non traitées (**a**, panneau du milieu), alors que sous l'effet des FTI ce pic est très réduit et la progérine apparaît géranylgéranylée et phosphorylée (**b**, panneau du bas). Après traitement pravastatin+zolédronate, la progérine non modifiée est la forme prédominante.

Légende de la figure 8: Analyse par spectrométrie de masse de protéines extraites de l'enveloppe nucléaire de fibroblastes non traitées (**a**), ou traitées par le mélange pravastatine+zolédronate (1 µM chacun, **b**) provenant de souris *Zmpste24*^{*-*/}*⁻.* La portion des spectres correspondant aux protéines non modifiées, farnésylées et géranylgéranylées est montrée en haut, au milieu et en bas de la figure. Chaque pic correspond au peptide tryptique de la progérine et est marqué avec la masse monoisotopique mesurée dans l'expérience et avec la masse théorique (entre parenthèses). Le numéro des résidus d'acide aminés est noté en bleu. La séquence des peptides et leur masse sont notées en rouge.

Sur cette figure, on remarque que la prélamine A est majoritairement farnésylée (F) et carboxyméthylée (Cm) dans les cellules non traitées (**a**, panneau du milieu), alors les formes non modifiées ou géranylgéranylées ne sont pas détectées. Après traitement par pravastatin+zolédronate, les peptides prénylés ne sont plus détectables et la forme non modifiée de la prélamine A est prédominante (b, panneau du haut).

### Le traitement par pravastatine+zolédronate corrige les anomalies nucléaire de cellules de patients progéria et de souris Zmpste24^{-/-} en culture, et restaure en partie les mécanismes de réparation des lésions de l'ADN induites par irradiation X (Figure 10, 11, 12 et 13).

Le traitement pravastatine+zolédronate provoque l'apparition de prélamine A dans le noyau de cellules témoins (Fig. 10a), comme dans le noyau de cellules de patients progéria, mais avec une nette amélioration de la morphologie nucléaire dans ces dernières (Fig. 10b). L'analyse quantitative montre une augmentation des anomalies nucléaires dans les cellules de patients progéria avec le nombre de passages, nombre d'anomalies qui diminue sous l'effet du traitement pravastatine+zolédronate (Fig. 10c). Observées au microscope confocal, les cellules de patient progéria contiennent des agrégats de lamine A/C, des invaginations profondes de la face nucléoplasmique de l'enveloppe nucléaire dans le nucléoplasme (le réticulum nucléaire) marquées par des anticorps anti-calréticuline (Fig. 11a-f). Ces agrégats de lamine A/C sont absents de cellules de sujets témoins (Fig. 10j-l) et disparaissent des cellules de patients progéria sous l'effet du traitement pravastatine+zolédronate (Fig. 10g-i). La localisation de la lamine B1, un constituant farnésylé de la lamina nucléaire, est modifiée sous l'effet du traitement, ce qui confirme que le traitement bloque la prénylation des lamines.

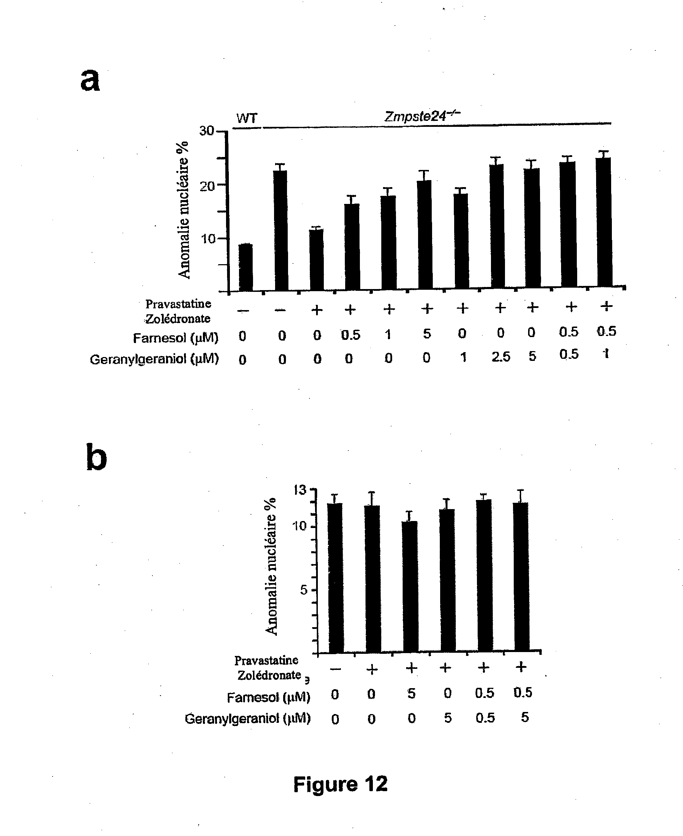

Nous avons vérifié que l'amélioration de la forme des noyaux par le traitement pravastatine+zolédronate est bien liée au blocage de la prénylation de la progérine, en incubant les cellules avec du farnésol et/ou du géranylgénaniol. La supplémentation des cellules par le farnésol et le géranylgéraniol permet aux cellules de synthétiser le farnésyl pyriphosphate et le géranylgéranyl pyrophosphate et donc prényler la progérine même en présence de pravastatine+zolédronate (Fig. 10d). Le farnésol abolit l'effet du traitement pravastatine+zolédronate, ce qui est une autre preuve que l'effet du traitement passe par l'inhibition de la synthèse du farnésyl pyrophosphate. Il est à noter que le géranylgéraniol bloque aussi l'effet du traitement, ce qui prouve que la forme géranylgéranylée de la progérine est elle aussi toxique pour les cellules (Fig. 10d). Les mêmes effets sont observés dans les cellules *Zmpste24*^{*-*/*-*} (Fig. 12a) ce qui suggère que les données concernant la progérine peuvent être étendues à la prélamine A, la protéine accumulée dans les cellules *Zmpste24*^{*-*/}*⁻.* Ni le farnésol, ni le géranylgeraniol ne produisent d'effet sur des fibroblastes témoins, ce qui élimine l'éventualité d'un artefact induit par ces molécules (Fig. 12b).

Enfin, le traitement pravastatine+zolédronate provoque une réduction du nombre de foci de l'histone H2AX phosphorylée, foci directement corrélés avec le nombre de cassures double brin de l'ADN non réparées (Fig. 13).

En conclusion, les données acquises *in vitro* montrent que la combinaison pravastatine+zolédronate inhibe partiellement la farnésylation et la géranylgéranylation, et provoque les modifications attendues de localisation au niveau de la lamina et la redistribution dans le nucléoplasme, de la prélamine A et de la progérine non prénylées dans les cellules *Zmpste24*^{*-*/*-*} et de patients progéria. De plus, la diminution de la quantité de la progérine farnésylée au niveau de la lamina et sa relocalisation dans le nucléoplasme explique les effets bénéfiques du traitement sur les cellules des patients progéria.

Légende de la figure 10 : Effet synergique de la combinaison pravastatine+zolédronate sur l'accumulation de prélamine A dans des cellules témoins et de patients progéria.
(**a**) Détection immunocytochimique de la lamine A/C et de la prélamine A dans des fibroblastes humains normaux, non traités, traités par pravastatine (60 µM, 12 h), par zolédronate (60 µM, 6 h) seuls ou associés.
(**b**) Détection immunocytochimique de la lamine A/C et de la prélamine A dans des fibroblastes humains normaux et de patients progéria traités pendant 24 h par la combinaison pravastatine+zolédronate (1 µM chacun).
(c) Analyse quantitative de l'effet du traitement pravastatine+zolédronate (1 µM chacun) sur la morphologie nucléaire de cellules de patients progéria. Les cellules traitées ou non ont été immunomarquées avec un anticorps anti-lamine A/C aux passages 8 (p8), 13 (p13) et 20 (p20). Les flèches blanches montrent les noyaux anormaux. (**d**) Analyse quantitative de l'effet du traitement pravastatine+zolédronate (1 µM chacun) sur la morphologie nucléaire de cellules de patients progéria en présence de farnésol, de géranylgéraniol ou des deux composés. Barres d'erreur = moyenne ± erreur standard de la moyenne. Barre d'échelle = 10 µm.

Légende de la figure 11: Le traitement par pravastatine+zolédronate corrige la morphologie nucléaire et induit une relocalisation partielle des isoformes de la lamine A/C et de la lamine B1 de la lamina nucléaire dans le nucléoplasme, dans des cellules de patients progéria.
(**A**) la colocalisation de la lamine A/C et de la calréticuline dans ces cellules progéria traitées ou non. Immunofluorescence et microscopie confocale (Leica TCS SP5, pile tridimensionnelle d'images 2048 x 2048 pixels, pas de 0,2 µm, moyenne de 3 lignes, accumulation de 3 images, zoom x 1,7). Les images **a** à **c** de chaque panneau sont des projections de l'intensité moyenne de 27 images de la pile et montrent les tubules du réticulum nucléaire marqués par la calréticuline dans les cellules progéria incubées avec le PBS. Images **d** à **1** : coupes confocales isolées de 0,2 µm d'épaisseur. Le traitement pravastatine+zolédronate corrige la forme des noyaux des cellules progéria, diminue le nombre des tubules du réticulum nucléaire **(g)** et l'épaisseur de la lamina nucléaire **(h).**
(B) Colocalisation de la lamine B1 et de la calréticuline. Le traitement pravastatine+zolédronate augmente le signal de marquage nucléoplasmique par la lamine B1, ce qui indique que la farnésylation de cette protéine est en partie inhibée. Barre d'échelle = 5 µm.

Légende de la figure 12: Les précurseurs du farnésyl-pyrophosphate et du géranyl-géranyl pyrophosphate abolissent l'effet du traitement pravastatine+zolédronate dans les cellules de souris *Zmpste24*^{-/-} en culture.

Quantification de l'effet de la pravastatine (**1** µM) et du zolédronate (1 µM) associés ou non sur la morphologie nucléaire de cellules de souris *Zmpste24*^{*-*/*-*} **(a)** et de souris témoins **(b)** en culture, en présence de farnésol, de géranylgéraniol ou des deux molécules.

Farnésol, géranylgéraniol seuls ou associés abolissent l'effet du traitement pravastatine+zolédronate sur la morphologie nucléaire de cellules *Zmpste24*^{*-*/}*⁻.*

Légende de la figure 13: Le traitement pravastatine+zolédronate corrige en partie les anomalies de la réparation des cassures double-brin (DSB) de l'ADN dans les cellules de patients progéria.

Des fibroblastes témoins et de patients progéria ont été incubés avec le mélange pravastatine+zolédonate (1 µM chacun) ou avec le PBS et ont été irradiés avec des rayons X (2 Gy). Immunodétection des foci de l'histone H2AX phosphorylée détectés 24 h après irradiation, foci correspondant aux cassures double brin non réparées (images du haut). Marquage nucléaire du DAPI (images du bas).

Courbes du bas : évolution du nombre de foci d'histone H2AX phosphorylée en fonction du temps après irradiation dans les cellules témoins (carré plein) et les cellules progéria (cercle vide) incubées avec le PBS ou traités avec pravastatine+zolédronate. Chaque courbe représente la moyenne ± erreur standard de la moyenne d'au moins 3 expériences.

### Le traitement associant pravastatine+zolédronate améliore le phénotype progéroïde de souris Zmpste24^{-/-} (Figures 14, 15 et 16) :

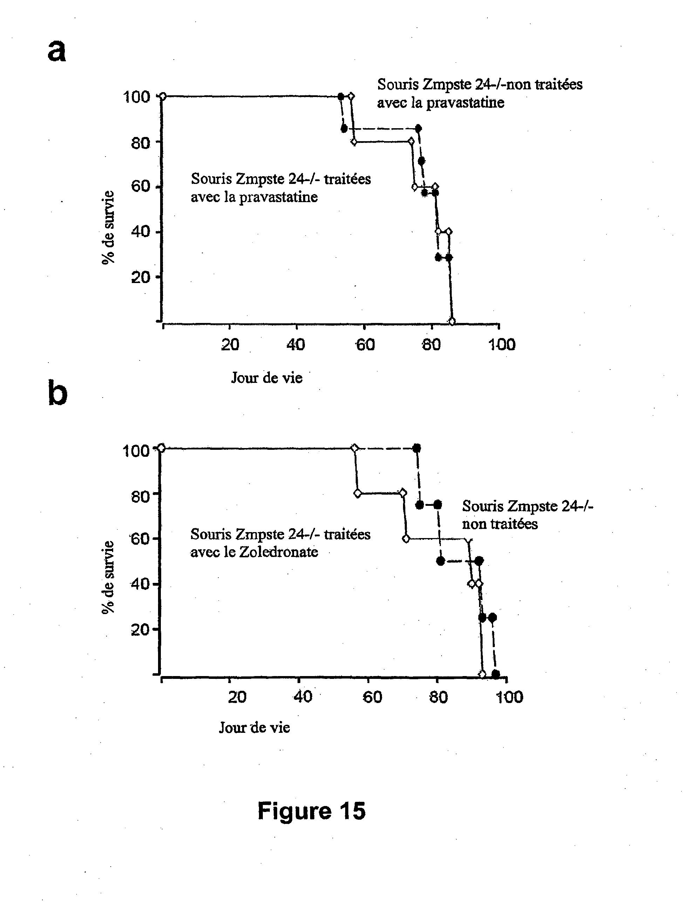

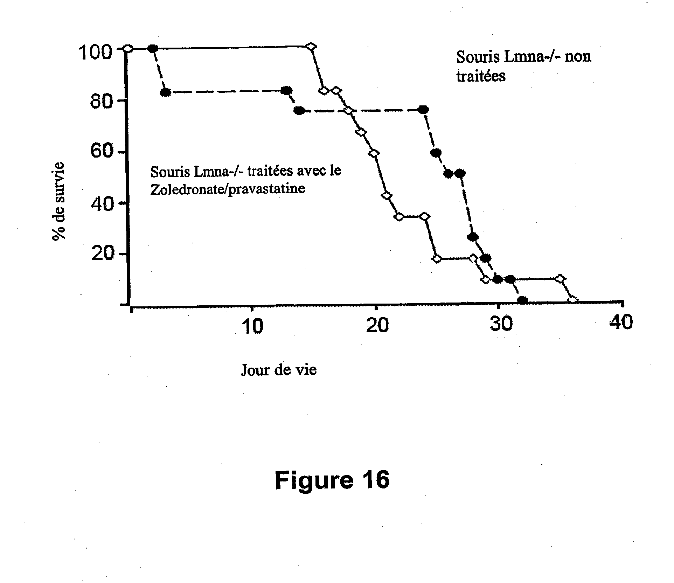

Les souris *Zmpste24*^{*-*/*-*} et les souris témoins ont été traitées quotidiennement avec la pravastatine, le zolédronate ou la combinaison des deux drogues à une dose qui avait été montrée précédemment comme étant dépourvue de toxicité chez la souris. Comme déjà observé pour les cellules de patient progéria, chacune des drogues isolément, pravastatine ou zolédronate, n'améliore pas la durée de vie des souris *Zmpste24*^{*-*/*-*} (Fig. 15). Au contraire, la combinaison des deux drogues améliore de manière significative le phénotype progéroïde des souris *Zmpste24*^{*-*/}*⁻ :* le traitement améliore la prise de poids, augmente la quantité de graisse sous-cutanée, réduit l'importance de la cyphose et de l'alopécie et augmente la durée de vie. La valeur moyenne de survie passe de 101 à 179 jours et la survie maximum passe de 151 à 222 jours (P < 0,001, Fig. 14c). Il est à remarquer que tous les signes phénotypiques corrigés par le traitement chez les souris sont aussi les caractéristiques de la progéria chez l'homme. Le traitement combiné corrige la diminution de la densité osseuse qui est l'une des caractéristiques des souris *Zmpste24*^{*-*/*-*} et de patients atteints de progéria ou d'un syndrome progéroïde apparenté. La microtomographie osseuse informatisée montre une augmentation de la minéralisation de l'os et de l'épaisseur de la corticale tibiale chez les souris traitées (Fig. 14d). De plus, la quantification des anomalies morphologiques nucléaires dans le foie de souris *Zmpste24*^{+/+} *, Zmpste24*^{-/-} et *Zmpste24*^{-/-} traitées montre que le traitement pravastatine+zolédronate normalise la forme des noyaux de cellules *Zmpste24*^{-/-} (Fig. 14e). Le traitement corrige en outre l'augmentation de la transcription des gènes cibles de la protéine p53, augmentation qui avait été décrite dans les cellules de souris *Zmpste24*^{-/-} (Varela et al., 2005) (Fig. 14f). Enfin, nous avons recherché si le traitement pouvait avoir un effet sur les souris *Lmna*^{-/-} qui ne peuvent pas accumuler de prélamine A. Le traitement pravastatine+zolédronate n'a aucun effet sur la durée de vie de ces souris (Fig. 16), ce qui est une preuve supplémentaire que ce traitement ne peut agir que chez des souris qui accumulent de la prélamine A farnésylée à l'enveloppe nucléaire.

Légende de la figure 14 : Le traitement pravastatine+zolédronate améliore le phénotype progéroïde de souris Zmpste24^{-/-} :

**(a)** Photographies représentatives de souris âgées de 3 mois *Zmpste24*^{+/+}, *Zmpste24*^{-/-} et *Zmpste24*^{-/-} traitées par la combinaison pravastatine (100 mg/kg/jour) et zolédronate (100 mg/kg/jour). Barre d'échelle = 1 cm. **(b)** Poids de souris âgées de 3 mois *Zmpste24*^{+/+} (n = 12), *Zmpste24*^{-/-} (n = 13) et *Zmpste24*^{-/-} traitées (n = 15). **(c)** Courbes de Kaplan-Meier montrant une augmentation significative de la dure de vie des souris *Zmpste24*^{-/-} traitées (n = 15) comparée à celle de souris non traitées (n = 13). **(d)** Représentation tridimensionnelle par microtomographie informatisée du tibia de souris *Zmpste24*^{-/-} traitée et non traitée (image du haut). Le panneau du bas représente le volume osseux relatif (volume du tissu osseux/volume du tibia) et le nombre de trabécules osseux dans les souris *Zmpste24*^{-/-} non traitées (n = 6) et traitées (n = 5). **(e)** Quantification des anomalies nucléaires des hépatocytes de souris *Zmpste24*^{+/+}, *Zmpste24*^{-/-} et *Zmpste24*^{-/-} traitées. Les flèches blanches montrent les noyaux anormaux. Barre d'échelle = 10 µm. **(f)** Expression relative des gènes cibles de la p53 dans le foie et le coeur de souris *Zmpste24*^{+/+}, *Zmpste24*^{-/-} et *Zmpste24*^{-/-} traitées, analysée par RT-PCR quantitative. * P < 0,05 ; ** P < 0,01 ; *** P < 0,001. Les barres d'erreur représentent la moyenne ± erreur standard de la moyenne.

Légende de la figure 15: Ni la pravastatine seule, ni le zolédronate seul n'augmente la durée de vie des souris Zmpste24^{-/-} :

Courbes de Kaplan-Meier montrant que la pravastine seule (n = 5) **(a),** et le zolédronate seul (n = 5) **(b)** ne corrigent pas l'a durée de vie des souris *Zmpste24*^{-/-}traitées (diamant vide) et non traitées (cercles pleins, n = 11).

Légende de la figure 16: Le traitement pravastatine+zolédronate ne corrige pas la durée de vie de souris *Lmna*^{-/-}:

Courbes de Kaplan-Meier montrant que durée de vie de souris *Lmna*^{-/-} traitées par pravastine+zolédronate (n= 12, diamant vide), comparée à celle de souris non traitées (cercles pleins, n = 11). Le traitement pravastatine+zolédronate est sans effet sur des souris dépourvues de lamine A/C.

### Résumé/conclusion/perspectives

Plusieurs syndromes progéroïdes humains, dont la progéria de Hutchinson-Gilford, sont causés par l'accumulation au niveau de l'enveloppe nucléaire d'une forme farnésylée de prélamine A délétée (progérine) ou non. La progérine est aussi produite au cours du vieillissement physiologique. Des études précédentes menées avec des cellules de patients atteints de progéria ont montré que les inhibiteurs de la farnésyltransférase (FTI) améliorent la morphologie des noyaux, suggérant que ces inhibiteurs pourraient représenter un traitement pour ces syndromes dévastateurs.

Les inventeurs montrent ici que la prélamine A et la progérine subissent une prénylation alternative par la géranylgéranyltransférase lorsque la farnésyltransférase est inhibée, ce qui pourrait expliquer la faible efficacité des FTI pour améliorer le phénotype de modèles murins de ces syndromes progéroïdes.

Ils montrent aussi que la combinaison d'une statine et d'un aminobiphosphonate inhibe de manière efficace à la fois la farnésylation et la géranylgéranylation de la prélamine A et de la progérine, améliore de manière significative le phénotype de vieillissement de souris chez lesquelles a été inactivée le gène codant la métalloprotéase Zmpste24 impliquée dans la maturation de la prélamine A. L'amélioration du phénotype inclut celle de la courbe de croissance, du poids, la lipodystrophie, la chute des poils et les anomalies osseuses.

De plus, la durée de vie de ces souris est augmentée de manière substantielle.

Ces données ouvrent une nouvelle approche thérapeutique des syndromes progéroïdes humains avec accumulation de protéines prénylées à l'enveloppe nucléaire.

Le traitement pravastatine+aminobiphosphonate est en cours d'application à Marseille pour les 3 années à venir sur des enfants atteints de progéria dans le cadre d'un essai thérapeutique européen (15 enfants) placé sous la responsabilité du Pr. Nicolas Lévy, financé par le Ministère de la Santé (PHRC 2008) et l'Association Française contre les Myopathies (AFM) et qui a reçu l'autorisation de l'AFSSAPS et du CCP Sud-Méditerranée.

Le même traitement va être prochainement donné à Rome sous la responsabilité du Pr. Giuseppe Novelli à des patients atteints de dysplasie acromandibulaire, un autre syndrome progéroïde avec accumulation de prélamine A farnésylée.

### Liste des références

(1) Basso AD, Kirschmeier P, Bishop WR. Farnesyl transferase inhibitors. J Lipid Res 47:15-31, 2006.
(2) Biamonti G, Giacca M, Perini G, Contreas G, Zentilin L, Weighardt F, Guerra M, Della Valle G, Saccone S, Riva S et al. The gene for a novel human lamin maps at a highly transcribed locus of chromosome 19 which replicates at the onset of S-phase. Mol Cell Biol 12:3499-3506, 1992.
(3) Bishop WR, Kirschmeier P, Baun C. Farnesyl transferase inhibitors: mechanism of action, translational studies and clinical evaluation. Cancer Biol Ther 2:S96-104, 2003.
(4) Broers JL, Hutchinson CJ, Ramaekers FC. Laminopathies. J Pathol 204:478-488, 2004.
(5) Broers JLV, Ramaekers FCS, Bonne G, Ben Yaou R, Hutchinson CJ. Nuclear lamins: laminopathies and their role in premature aging. Physiol Rev 86:967-1008, 2006.
(6) Capell BC, Erdos MR, Madigan JP, Fiordalisi JJ, Varga R, Conneely KN, Gordon LB, Der CJ, Cox AD, Collins FS. Inhibiting farnesylation of progerin prevents the characteristic nuclear blebbing of Hutchinson-Gilford progeria syndrome. Proc Natl Acad Sci USA 102:12879-12884, 2005.
(7) De Sandre-Giovannoli A, Bernard R, Cau P, Navarro C, Amiel J, Boccaccio I, Lyonnet S, Stewart CL, Munnich A, Le Merrer M, Levy N. Lamin A truncation in Hutchinson-Gilford progeria. Science 300:2055, 2003.
(8) Demyanets S, Kaun C, Pfaffenberger S, Philipp J. Hohensinner PJ, Rega G, Pammer J, Maurer G, Huber K, Wojta J. Hydroxymethylglutaryl coenzyme A reductase inhibitors induce apoptosis in human cardiac myocytes in vitro. Biochem Pharmacol 71:1324-1330, 2006.
(9) Duque G, Rivas D. Age-related changes in Lamin A/C expression in the osteoarticular system: laminopathies as a potentiel new aging mechanism. Mech Aging Dev 127:378-383, 2006.
(10) Efuet ET, Keyomarsi K. Farnesyl and geranylgeranyl transferase inhibitors induce G1 arrest by targeting the proteasome. Cancer Res 66:1040-1051, 2006.
(11) Eriksson M, Brown WT, Gordon LB, Glynn MW, Singer J, Scott L, Erdos MR, Robbins CM, Moses TY, Berglund P, Dutra A, Pak E, Durkin S, Csoka AB, Boehnke M, Glover TW, Collins FS. Recurrent de novo point mutation in lamin A cause Hutchinson-Gilford progeria syndrome. Nature 423:293-298, 2003.
(12) Evans M, Rees A. The myotoxicity of statins. Cur Op Lipid, 13:415-420, 2002.
(13) Flint OP, Masters BA, Gregg RE, Durham SK. HMG CoA reductase inhibitor-induced myotoxicity: pravastatin and lovastatin inhibit the geranylgeranylation of low-molecular-weight proteins in neonatal rat muscle cell culture. Tox Appl Pharmacol 145:99-110, 1997.
(14) Fong LG, Frost D, Meta M, Qiao X, Yang SH, Coffinier C, Young SG. A protein farnesylfransferase inhibitor ameliorates disease in a mouse model of progeria. Science,311 : 1621-1623, 2006.
(15) Fong LG, Ng JK, Lammerding J, Vickers TA, Meta M, Coté N, Gavino B, Qiao X, Chang SY, Young SR, Yang SH, Stewart CL, Lee RT, Bennett CF, Bergo MO, Young SG. Prelamin A and Lamin A appear to be dispensable in the nuclear lamina. J Clin Invest 116:743-752,2006.
(16) Fong LG, Ng JK, Meta M, Cote N, Yang SH, Stewart CL, Sullivan T, Burghardt A, Majumdar S, Reue K, Bergo MO, Young SG. Heterozygosity for Lmna deficiency eliminates the progeria-like phenotypes in Zmpste24-deficient mice. Proc Natl Acad Sci USA 101:18111-18116, 2004.
(17) Glynn MW, Glover TW. Incomplète processing of mutant lamin A in Hutchinson-Gilford progeria leads to nuclear abnormalities, which are reversed by farnesyltransferase inhibition. Hum Mol Genet 14:2959-2969,2005.
(18) Goldman RD, Shumaker DK, Erdos MR, Eriksson M, Goldman AE, Gordon LB, Gruenbaum Y, Khuon S, Mendez M, Varga R, Collins FS. Accumulation of mutant lamin A causes progressive changes in nuclear architecture in Hutchinson-Gilford progeria syndrome. Proc Natl Acad Sci USA 101:8963-8968, 2004.
(19) Gruenbaum Y, Margalit A, Goldman RD, Shumaker DK, Wilson KL. The nuclear lamina comes of age. Nat Mol Cell Biol 6:21-31, 2005.
(20) Hampton R, Dimster-Denk D, Rine J. The biology of HMG-CoA reductase: the pros of contra-regulation. Trends Biochem Sci 21:140-145, 1996.
(21) Harborth J, Elbashir SM, Bechert K, Tuschl T, Weber K. Identification of essential genes in cultured mammalian cells using small interfering RNAs. J Cell Sci 114:4557-4565, 2001.
(22) Hegele RA, Cao H, Liu DM, Costain GA, Charlton-Menys V, Rodger NW, Durrington PN. Sequencing of reannotated LMNB2 gene reveals novel mutations in patients with acquired partial lipodystrophy. Am J Hum Genet 79:383-389, 2006.
(23) Hildebrand T, Ruegsegger P. A new method for the model independent assessment of thickness in three dimensional images. J Microsc 185:67-75, 1997.
(24) Hoffmann GF, Charpentier C, Mayatepek E, Mancini J, Leichsenring M, Gibson KM, Divry P, Hrebicek M, Lehnert W, Sartor K. Clinical and biochemical phenotype in 11 patients with mevalonic aciduria. Pediatrics 91:915-921, 1993.
(25) Huang S, Chen L, Libina N, Janes J, Martin GM, Campisi J, Oshima J._Correction of cellular phenotypes of Hutchinson-Gilford Progeria cells by RNA interference. Hum Genet. 2005 Oct 6;: 1-7
(26) Hutchinson CJ, Worman HJ. A-type lamins: guardians of the soma? Nat Cell Biol 6:1062-1067, 2004.
(27) Kusuyama T, Omura T, Nishiya D, Enomoto S, Matsumoto R, Murata T, Takeuchi K, Yoshikawa J, Yoshiyama M. The effects of HMG-CoA reductase inhibitor on vascular progenitor cells. J Pharmacol Sci 1001:344-349, 2006.
(28) Leung KF, Baron R, Seabra MC. Geranylgeranylation of Rab GTPases. J Lipid Res 47:467-475, 2006.
(29) Lévy N, Cau P. Anomalies du noyau et maladies. Pour la Science 313 :2-7, 2003.
(30) Lin F, Worman HJ. Structural organization of the human gene (LMNB1) encoding nuclear lamin B1. Genomics 27:230-236, 1995.
(31) Lin F, Worman HJ. Structural organization of the human gene encoding nuclear lamin A and nuclear lamin C. J Biol Chem 268:16321-16326, 1993.
(32) Liu Y, Wang Y, Rusinol AE, Sinensky MS, Liu J, Shell SM, Zou Y. Involvement of xeroderma pigmentosum group A (XPA) in progeria arising from defective maturation of prelamin A. FASEB J. 2007 Sep 11;
(33) Mattout A, Dechat T, Adam SA, Goldman RD, Gruenbaum Y. Nuclear lamins, diseases and aging. Cur Op Cell Biol 18:335-341, 2006.
(34) McClintock D, Ratner D, Lokuge M, Owens DM, Gordon LB, Collins FS, Djhabali K. The mutatnt form of lamin A that causes Hutchinson-Gilford progeria is a biomarker of cellular aging in human skin. PloS One, 2007, 2, e1269
(35) Navarro CL, Cadinanos J, De Sandre-Giovannoli A, Bernard R, Courrier S, Boccaccio I, Boyer A, Kleijer WJ, Wagner A, Giuliano F, Beemer FA, Freije JM, Cau P, Hennekam RC, Lopez-Otin C, Badens C, Levy N. Loss of ZMPSTE24 (FACE-1) causes autosomal recessive restrictive dermopathy and accumulation of Lamin A precursors. Hum Mol Genet 14:1503-1513, 2005.
(36) Navarro CL, De Sandre-Giovannoli A, Bernard R, Boccaccio I, Boyer A, Genevieve D, Hadj-Rabia S, Gaudy-Marqueste C, Smitt HS, Vabres P, Faivre L, Verloes A, Van Essen T, Flori E, Hennekam R, Beemer FA, Laurent N, Le Merrer M, Cau P, Levy N. Lamin A and ZMPSTE24 (FACE-1) defects cause nuclear disorganization and identify restrictive dermopathy as a lethal neonatal laminopathy. Hum Mol Genet 13:2493-2503, 2004.
(37) Padiath QS, Saigoh K, Schiffmann R, Asahara H, Yamada T, Koeppen A, Hogan K, Ptacek LJ, Fu YH. Lamin B1 duplications cause autosomal dominant leukodystrophy. Nature Genet 38:1114-1123,2006.
(38) Pendas AM, Zhou Z, Cadinanos J, Freije JM, Wang J, Hultenby K, Astudillo A, Wernerson A, Rodriguez F, Tryggvason K, Lopez-Otin C. Defective prelamin A processing and muscular and adipocyte alterations in Zmpste24 metalloproteinase-deficient mice. Nat Genet 31:94-99, 2002.
(39) Reid TS, Terry KL, Casey PJ, Beese LS. Crystallographic analysis of CaaX prenyltransferases complexed with substrates defines rules of protein substrate selectivity. J Mol Biol 343:417-433, 2004.
(40) Scaffidi P, Misteli T. Lamin A-dependent nuclear defects in human aging. Sciencexpress, 27 avril 2006.
(41) Scaffidi P, Misteli T. Reversal of the cellular phenotype in the premature aging disease Hutchinson-Gilford progeria syndrome. Nat Med 11:440-445, 2005.
(42) Scaffidi P, Misteli T. Reversal of the cellular phenotype in the premature aging disease Hutchinson-Gilford progeria syndrome. Nature Med 11:440-445, 2005.
(43) Shelton KR, Egle PM, Cochran DL. Nuclear envelope proteins: identification of lamin B subtypes. Biochem Biophys Res Comm 103:975-981, 1981.
(44) Shumaker DK, Kuczmarski ER, Goldman RD. The nucleoskeleton: lamins an actin are major players in essential nuclear functions. Curr Op Cell Biol 15:358-366, 2003.
(45) Stewart C, Burke B. Teratocarcinoma stem cells and early mouse embryos contain only a single major lamin polypeptide closely resembling lamin B. Cell 51:383-392, 1987.
(46) Takedaa M, Noshiroa R, Onozatob ML, Tojob A, Hasannejada H, Huangc XL, Narikawac S, Endoua H. Evidence for a role of human organic anion transporters in the muscular side effects of HMG-CoA reductase inhibitors. Eur J Pharm 483 :133-138, 2004.
(47) Toth JI, Yang SH, Qiao X, Beigneux AP, Gelb MH, Moulson CL, Miner JH, Young SG, Fong LG. Blocking protein farnesyltransferase improves nuclear shape in fibroblasts from humans with progeroid syndromes. Proc Natl Acad Sci USA 102:12873-12878, 2005.
(48) Tsai MY, Wang S, Heidinger JM, Shumaker DK, Adam SA, Goldman RD, Zheng Y. A mitotic lamin B matrix induced by RanGTP required for spindle assembly. Science 311:1887-1893, 2006.
(49) Varela I, Cadinanos J, Pendas AM, Gutierrez-Fernandez A, Folgueras AR, Sanchez LM, Zhou Z, Rodriguez FJ, Stewart CL, Vega JA, Tryggvason K, Freije JM, Lopez-Otin C. Accelerated ageing in mice deficient in Zmpste24 protease is linked to p53 signalling activation. Nature 437:564-568, 2005.
(50) Vergnes L, Peterfy M, Bergo MO, Young SG, Reue K. Lamin B1 is required for mouse development and nuclear integrity. Proc Natl Acad Sci USA 101:10428-10433, 2004.
(51) Winter-Vann AM, Casey PJ. Post-prenylation-processing enzymes as new targets in oncogenesis. Nat Rev Cancer 5:405-412, 2005.
(52) Wydner KL, McNeil JA, Lin F, Worman HJ, Lawrence JB. Chromosomal assignment of human nuclear envelope protein genes LMNA, LMNB1 and LBR by fluorescence in situ hybridization. Genomics 32:474-478, 1996.
(53) Young SG, Meta M, Yang SH, Fong LG. Prelamin A farnesylation and progeroid syndromes. J Biol Chem 281:39741-39745, 2006.
(54) Zastrow MS, Vlcek S, Wilson KL. Proteins that bind A-type lamins: integrating isolated clues. J Cell Sci 117:979-987, 2004.
(55) Varela, I. Pereira, S. Ugalde, A. P. Navarro, C. L. Suarez, M. F. Cau, P. Cadinanos, J. Osorio, F. G. Foray, N. Cobo, J. de Carlos, F. Levy, N. Freije, J. M. Lopez-Otin, C. Combined treatment with statins and aminobisphosphonates extends longevity in a mouse model of human premature aging. Nature Medicine 14: 767-772, 2008
(56) Sullivan, T. Escalante-Alcalde, D. Bhatt, H. Anver, M. Bhat, N. Nagashima, K. Stewart, C. L. Burke, B. Loss of A-type lamin expression compromises nuclear envelope integrity leading to muscular dystrophy. J. Cell Biol. 147: 913-920, 1999

## Revendications

1. Composition topique dermatologique comprenant :
- au moins un inhibiteur de l'hydroxyméthylglutaryl-coenzyme A (HMG-CoA) réductase, et
- au moins un inhibiteur de la farnésyl-pyrophosphate synthase,
pour son utilisation dans le traitement de désordres cutanés et/ou pileux choisis dans le groupe comprenant le vieillissement cutané précoce pathologique et/ou le vieillissement myo-lipo-cutané précoce pathologique.

2. Composition selon la revendication 1 dans laquelle l'inhibiteur de HMG-CoA réductase est une molécule de la famille des statines ou l'un de ses sels physiologiquement acceptables.

3. Composition selon la revendication 2, dans laquelle l'inhibiteur de HMG-CoA réductase est une statine hydrosoluble.

4. Composition selon la revendication 2, dans laquelle l'inhibiteur de HMG-CoA réductase est une statine liposoluble.

5. Composition selon l'une quelconque des revendications 1 à 4 dans laquelle l'inhibiteur de HMG-CoA réductase est choisi dans le groupe comprenant l'atorvastatine, la simvastatine, la pravastatine, la rivastatine, la mévastatine (ou compactine), la fluindostatine, la vélostatine, la fluvastatine, la dalvastatine, la cérivastatine, la pentostatine, la rosuvastatine, la lovastatine, la pitavastatine et leurs sels physiologiquement acceptables.

6. Composition selon l'une quelconque des revendications 1 à 5, dans laquelle l'inhibiteur de la farnésyl-pyrophosphate synthase est choisi dans le groupe comprenant une molécule de la famille des aminobiphosphonates (NBP) ou l'un de ses sels physiologiquement acceptables.

7. Composition selon la revendication 6, dans laquelle l'aminobiphosphonate peut être choisi parmi :
- l'acide alendronique ou sa forme ionique, l'alendronate ;
- l'acide clodronique ou sa forme ionique, le clodronate ;
- l'acide étidronique ou sa forme ionique, l'étidronate ;
- l'acide ibandronique, ou sa forme ionique, l'ibandronate;
- l'acide médronique ou sa forme ionique, le médronate ;
- l'acide néridronique ou sa forme ionique, le néridronate;
- l'acide olpadronique ou sa forme ionique, l'olpadronate ;
- l'acide pamidronique ou sa forme ionique, le pamidronate;
- l'acide risédronique ou sa forme ionique, le risédronate;
- l'acide tiludronique ou sa forme ionique, le tiludronate ;
- l'acide zolédronique (ou zolendronique) ou sa forme ionique le zolédronate (ou zolendronate) ;
- l'acide 4-N,N-diméthylaminométhane diphosphonique ou sa forme ionique le diméthylaminométhanediphosphonate ;
- l'α-amino-(4-hydroxybenzylidène) diphosphonate.

8. Composition selon l'une quelconque des revendications 1 à 7, dans laquelle l'inhibiteur de la farnésyl-pyrophosphate synthase est l'acide zolédronique ou sa forme ionique, le zolédronate.

9. Composition selon l'une quelconque des revendications 1 à 8, dans laquelle la quantité d'inhibiteur de la farnésyl-pyrophosphate synthase est comprise entre 0,001 à 0,050 pour cent en poids de la composition cosmétique et/ou dermatologique.

10. Composition selon l'une quelconque des revendications 1 à 9, dans laquelle la quantité d'inhibiteur de la HMG-CoA réductase est comprise entre 0,010 à 0,100 pour cent en poids de la composition dermatologique.

11. Composition selon l'une quelconque des revendications 1 à 10, dans laquelle la somme de la quantité d'inhibiteur de la HMG-CoA réductase et de la quantité d'inhibiteur de la farnésyl-pyrophosphate synthase est comprise entre 0,011 et 0,150 pour cent en poids de la composition dermatologique.

12. Utilisation cosmétique non thérapeutique d'une composition cosmétique comprenant :
- au moins un inhibiteur de l'hydroxyméthylglutaryl-coenzyme A (HMG-CoA) réductase, et
- au moins un inhibiteur de la farnésyl-pyrophosphate synthase
pour le traitement de désordres cutanés et/ou pileux choisis dans le groupe comprenant le vieillissement cutané et/ou la lipodystrophie.

13. Utilisation selon la revendication 12 dans laquelle l'inhibiteur de HMG-CoA réductase est une molécule de la famille des statines ou l'un de ses sels physiologiquement acceptables.

14. Utilisation selon la revendication 13, dans laquelle l'inhibiteur de HMG-CoA réductase est une statine hydrosoluble.

15. Utilisation selon la revendication 13, dans laquelle l'inhibiteur de HMG-CoA réductase est une statine liposoluble.

16. Utilisation selon la revendication 13, dans laquelle l'inhibiteur de HMG-CoA réductase est choisi dans le groupe comprenant l'atorvastatine, la simvastatine, la pravastatine, la rivastatine, la mévastatine (ou compactine), la fluindostatine, la vélostatine, la fluvastatine, la dalvastatine, la cérivastatine, la pentostatine, la rosuvastatine, la lovastatine, la pitavastatine et leurs sels physiologiquement acceptables.

17. Utilisation selon l'une quelconque des revendications 12 à 16, dans laquelle l'inhibiteur de la farnésyl-pyrophosphate synthase est choisi dans le groupe comprenant une molécule de la famille des aminobiphosphonates (NBP) ou l'un de ses sels physiologiquement acceptables.

18. Utilisation selon la revendication 17, dans laquelle l'aminobiphosphonate peut être choisi parmi :
- l'acide alendronique ou sa forme ionique, l'alendronate ;
- l'acide clodronique ou sa forme ionique, le clodronate ;
- l'acide étidronique ou sa forme ionique, l'étidronate ;
- l'acide ibandronique, ou sa forme ionique, l'ibandronate;
- l'acide médronique ou sa forme ionique, le médronate ;
- l'acide néridronique ou sa forme ionique, le néridronate;
- l'acide olpadronique ou sa forme ionique, l'olpadronate ;
- l'acide pamidronique ou sa forme ionique, le pamidronate;
- l'acide risédronique ou sa forme ionique, le risédronate;
- l'acide tiludronique ou sa forme ionique, le tiludronate ;
- l'acide zolédronique (ou zolendronique) ou sa forme ionique le zolédronate (ou zolendronate) ;
- l'acide 4-N,N-diméthylaminométhane diphosphonique ou sa forme ionique le diméthylaminométhanediphosphonate ;
- l'α-amino-(4-hydroxybenzylidène) diphosphonate.

19. Utilisation selon l'une quelconque des revendications 12 à 17, dans laquelle l'inhibiteur de la farnésyl-pyrophosphate synthase est l'acide zolédronique ou sa forme ionique, le zolédronate.

20. Utilisation selon l'une quelconque des revendications 12 à 19, dans laquelle la quantité d'inhibiteur de la farnésyl-pyrophosphate synthase est comprise entre 0,001 à 0,050 pour cent en poids de la composition cosmétique.

21. Utilisation selon l'une quelconque des revendications 12 à 20, dans laquelle la quantité d'inhibiteur de la HMG-CoA réductase est comprise entre 0,010 à 0,100 pour cent en poids de la composition cosmétique.

22. Utilisation selon l'une quelconque des revendications 12 à 21, dans laquelle la somme de la quantité d'inhibiteur de la HMG-CoA réductase et de la quantité d'inhibiteur de la farnésyl-pyrophosphate synthase est comprise entre 0,011 et 0,150 pour cent en poids de la composition cosmétique.

23. Utilisation selon l'une quelconque des revendications 12 à 21, dans laquelle la composition cosmétique comprend au moins un des composés choisis dans le groupe comprenant un tensioactif, un agent épaississant, un agent gélifiant, un conservateur, un agent humectant, un agent émulsifiant, du parfum, un silicone, un agent chélatant, un antioxydant, un agent fongicide, un agent antibactérien, un colorant cosmétique, un agent absorbant, un agent filmogène, un agent stabilisateur, un agent tampon, un filtre UV, un hydratant, un liant, un stabilisateur d'émulsion.

24. Utilisation selon l'une quelconque des revendications 12 à 23, dans laquelle la composition cosmétique est sous une forme d'une crème, d'un aérosol, d'un gel, d'un onguent, d'une poudre, d'une mousse, d'un lait, d'un complément alimentaire ou d'un produit administrable par voie orale.

## Patentansprüche

1. Topische dermatologische Zusammensetzung, die Folgendes umfasst:
- mindestens einen Hemmer der Hydroxymethylglutaryl-Coenzym-A-Reduktase (HMG-CoA-Reduktase) und
- mindestens einen Hemmer der Farnesylpyrophosphat-Synthase,
für deren Verwendung bei der Behandlung von Haut- und/oder Haarstörungen, die aus der Gruppe umfassend pathologische frühzeitige Hautalterung und/oder pathologische frühzeitige myolipokutane Alterung ausgewählt sind.

2. Zusammensetzung nach Anspruch 1, wobei der Hemmer der HMG-CoA-Reduktase ein Molekül der Familie von Statinen oder eines seiner physiologisch akzeptablen Salze ist.

3. Zusammensetzung nach Anspruch 2, wobei der Hemmer der HMG-CoA-Reduktase ein wasserlösliches Statin ist.

4. Zusammensetzung nach Anspruch 2, wobei der Hemmer der HMG-CoA-Reduktase ein fettlösliches Statin ist.

5. Zusammensetzung nach einem der Ansprüche 1 bis 4, wobei der Hemmer der HMG-CoA-Reduktase aus der Gruppe umfassend Atorvastatin, Simvastatin, Pravastatin, Rivastatin, Mevastatin (oder Compactin), Fluindostatin, Velostatin, Fluvastatin, Dalvastatin, Cerivastatin, Pentostatin, Rosuvastatin, Lovastatin, Pitavastatin und deren physiologisch akzeptable Salze ausgewählt ist.

6. Zusammensetzung nach einem der Ansprüche 1 bis 5, wobei der Hemmer der Farnesylpyrophosphat-Synthase aus der Gruppe umfassend ein Molekül der Familie von Aminobiphosphonaten (NBP) oder eines seiner physiologisch akzeptablen Salze ausgewählt ist.

7. Zusammensetzung nach Anspruch 6, wobei das Aminobiphosphonat aus folgenden ausgewählt sein kann:
- Alendronsäure oder ihrer ionischen Form, Alendronat;
- Clodronsäure oder ihrer ionischen Form, Clodronat;
- Etidronsäure oder ihrer ionischen Form, Etidronat;
- Ibandronsäure oder ihrer ionischen Form, Ibandronat;
- Medronsäure oder ihrer ionischen Form, Medronat;
- Neridronsäure oder ihrer ionischen Form, Neridronat;
- Olpadronsäure oder ihrer ionischen Form, Olpadronat;
- Pamidronsäure oder ihrer ionischen Form, Pamidronat;
- Risedronsäure oder ihrer ionischen Form, Risedronat;
- Tiludronsäure oder ihrer ionischen Form, Tiludronat;
- Zoledronsäure (oder Zolendronsäure) oder ihrer ionischen Form, Zoledronat (oder Zolendronat);
- 4-N,N-Dimethylaminomethandiphosphonsäure oder ihrer ionischen Form, Dimethylaminomethandiphosphonat;
- α-Amino-(4-hydroxybenzyliden)diphosphonat.

8. Zusammensetzung nach einem der Ansprüche 1 bis 7, wobei der Hemmer der Farnesylpyrophosphat-Synthase Zoledronsäure oder ihre ionische Form, Zoledronat, ist.

9. Zusammensetzung nach einem der Ansprüche 1 bis 8, wobei die Menge des Hemmers der Farnesylpyrophosphat-Synthase zwischen 0,001 und 0,050 Gewichtsprozent der kosmetischen und/oder dermatologischen Zusammensetzung liegt.

10. Zusammensetzung nach einem der Ansprüche 1 bis 9, wobei die Menge des Hemmers der HMG-CoA-Reduktase zwischen 0,010 und 0,100 Gewichtsprozent der dermatologischen Zusammensetzung liegt.

11. Zusammensetzung nach einem der Ansprüche 1 bis 10, wobei die Summe der Menge des Hemmers der HMG-CoA-Reduktase und der Menge des Hemmers der Farnesylpyrophosphat-Synthase zwischen 0,011 und 0,150 Gewichtsprozent der dermatologischen Zusammensetzung liegt.

12. Nichttherapeutische kosmetische Verwendung einer kosmetischen Zusammensetzung, die Folgendes umfasst:
- mindestens einen Hemmer der Hydroxymethylglutaryl-Coenzym-A-Reduktase (HMG-CoA-Reduktase) und
- mindestens einen Hemmer der Farnesylpyrophosphat-Synthase,
zur Behandlung von Haut- und/oder Haarstörungen, die aus der Gruppe umfassend Hautalterung und/oder Lipodystrophie ausgewählt sind.

13. Verwendung nach Anspruch 12, wobei der Hemmer der HMG-CoA-Reduktase ein Molekül der Familie von Statinen oder eines seiner physiologisch akzeptablen Salze ist.

14. Verwendung nach Anspruch 13, wobei der Hemmer der HMG-CoA-Reduktase ein wasserlösliches Statin ist.

15. Verwendung nach Anspruch 14, wobei der Hemmer der HMG-CoA-Reduktase ein fettlösliches Statin ist.

16. Verwendung nach Anspruch 13, wobei der Hemmer der HMG-CoA-Reduktase aus der Gruppe umfassend Atorvastatin, Simvastatin, Pravastatin, Rivastatin, Mevastatin (oder Compactin), Fluindostatin, Velostatin, Fluvastatin, Dalvastatin, Cerivastatin, Pentostatin, Rosuvastatin, Lovastatin, Pitavastatin und deren physiologisch akzeptable Salze ausgewählt ist.

17. Verwendung nach einem der Ansprüche 12 bis 16, wobei der Hemmer der Farnesylpyrophosphat-Synthase aus der Gruppe umfassend ein Molekül der Familie von Aminobiphosphonaten (NBP) oder eines seiner physiologisch akzeptablen Salze ausgewählt ist.

18. Verwendung nach Anspruch 17, wobei das Aminobiphosphonat aus folgenden ausgewählt sein kann:
- Alendronsäure oder ihrer ionischen Form, Alendronat;
- Clodronsäure oder ihrer ionischen Form, Clodronat;
- Etidronsäure oder ihrer ionischen Form, Etidronat;
- Ibandronsäure oder ihrer ionischen Form, Ibandronat;
- Medronsäure oder ihrer ionischen Form, Medronat;
- Neridronsäure oder ihrer ionischen Form, Neridronat;
- Olpadronsäure oder ihrer ionischen Form, Olpadronat;
- Pamidronsäure oder ihrer ionischen Form, Pamidronat;
- Risedronsäure oder ihrer ionischen Form, Risedronat;
- Tiludronsäure oder ihrer ionischen Form, Tiludronat;
- Zoledronsäure (oder Zolendronsäure) oder ihrer ionischen Form, Zoledronat (oder Zolendronat);
- 4-N,N-Dimethylaminomethandiphosphonsäure oder ihrer ionischen Form, Dimethylaminomethandiphosphonat;
- α-Amino-(4-hydroxybenzyliden)diphosphonat.

19. Verwendung nach einem der Ansprüche 12 bis 17, wobei der Hemmer der Farnesylpyrophosphat-Synthase Zoledronsäure oder ihre ionische Form, Zoledronat, ist.

20. Verwendung nach einem der Ansprüche 12 bis 19, wobei die Menge des Hemmers der Farnesylpyrophosphat-Synthase zwischen 0,001 und 0,050 Gewichtsprozent der kosmetischen Zusammensetzung liegt.

21. Verwendung nach einem der Ansprüche 12 bis 20, wobei die Menge des Hemmers der HMG-CoA-Reduktase zwischen 0,010 und 0,100 Gewichtsprozent der kosmetischen Zusammensetzung liegt.

22. Verwendung nach einem der Ansprüche 12 bis 21, wobei die Summe der Menge des Hemmers der HMG-CoA-Reduktase und der Menge des Hemmers der Farnesylpyrophosphat-Synthase zwischen 0,011 und 0,150 Gewichtsprozent der kosmetischen Zusammensetzung liegt.

23. Verwendung nach einem der Ansprüche 12 bis 21, wobei die kosmetische Zusammensetzung mindestens eine der Verbindungen umfasst, die aus der Gruppe umfassend ein Tensid, ein Verdickungsmittel, ein Geliermittel, ein Konservierungsmittel, ein Feuchthaltemittel, ein Emulgiermittel, Parfüm, ein Silikon, einen Chelatbildner, ein Antioxidationsmittel, ein Fungizid, ein antibakteriell wirkendes Mittel, einen kosmetischen Farbstoff, ein Absorptionsmittel, einen Filmbildner, ein Stabilisierungsmittel, ein Puffermittel, einen UV-Filter, einen Feuchtigkeitsspender, ein Bindemittel, einen Emulsionsstabilisator ausgewählt sind.

24. Verwendung nach einem der Ansprüche 12 bis 23, wobei die kosmetische Zusammensetzung in einer Form einer Creme, eines Sprays, eines Gels, einer Salbe, eines Puders, eines Schaums, einer Milch, eines Nahrungsergänzungsmittels oder eines oral verabreichbaren Produkts ist.

## Claims

1. Dermatological topic composition comprising:
- at least one inhibitor of hydroxymethylglutaryl-coenzyme A (HMG-CoA) reductase, and
- at least one inhibitor of farnesyl pyrophosphate synthase,
for its uses in the treatment of skin and/or hair disorders selected from the group comprising the pathological premature skin ageing and/or pathological premature myolipocutaneous ageing.

2. Composition according to claim 1, wherein the HMG-CoA reductase inhibitor is a molecule of the statins family or one of the physiologically acceptable salts thereof.

3. Composition according to claim 2, wherein the HMG-CoA reductase inhibitor is a water-soluble statin.

4. Composition according to claim 2, wherein the HMG-CoA reductase inhibitor is a lipid-soluble statin.

5. Composition according to any one of claim 1 to 4, wherein the HMG-CoA reductase inhibitor is selected from the group comprising atorvastatin, simvastatin, pravastatin, rivastatin, mevastatin (or compactin), fluindostatin, velostatin, fluvastatin, dalvastatin, cerivastatin, pentostatin, rosuvastatin, lovastatin, pitavastatin, or one of the physiologically acceptable salts thereof.

6. Composition according to any one claim 1 to 5, wherein the farnesylpyrophosphate synthase inhibitor is selected from the group comprising a molecule of the family of aminobiphosphonates (NBPs) or one of the physiologically acceptable salts thereof.

7. Composition according to claim 6, wherein the aminobiphosphonate can be selected among:
- alendronic acid or the ionic form thereof, alendronate;
- clodronic acid or the ionic form thereof, clodronate;
- etidronic acid or the ionic form thereof, etidronate;
- ibandronic acid or the ionic form thereof, ibandronate;
- medronic acid or the ionic form thereof, medronate;
- neridronic acid or the ionic form thereof, neridronate;
- olpadronic acid or the ionic form thereof, olpadronate;
- pamidronic acid or the ionic form thereof, pamidronate;
- risedronic acid or the ionic form thereof, risedronate;
- tiludronic acid or the ionic form thereof, tiludronate;
- zoledronic acid or the ionic form thereof, zoledronate;
- 4-N,N-dimethylaminomethane diphosphonic acid or the ionic form thereof, dimethylaminomethanediphosphonate;
- a-amino-(4-hydroxybenzylidene) diphosphonate.

8. Composition according to any one of claim 1 to 7, wherein the farnesylpyrophosphate synthase inhibitor is zoledronic acid or the ionic form thereof, zoledronate.

9. Composition according to any one of claim 1 to 8, wherein the quantity of farnesylpyrophosphate synthase inhibitor is from 0.001 to 0.050 percent by weight of the cosmetic and/or dermatological composition.

10. Composition according to any one of claim 1 to 9, wherein the quantity of HMG-CoA reductase inhibitor is from 0.010 to 0.100 percent by weight of the dermatological composition.

11. Composition according to any one of claim 1 to 10, wherein the sum of the quantity of HMG-CoA reductase inhibitor and quantity of farnesylpyrophosphate synthase inhibitor is from 0.011 to 0.150 percent by weight of the dermatological composition.

12. Non-therapeutic cosmetic use of a cosmetic composition comprising :
- at least one inhibitor of hydroxymethylglutaryl-coenzyme A (HMG-CoA) reductase, and
- at least one inhibitor of farnesyl pyrophosphate synthase,
for the treatment of skin and/or hair disorders selected from the group comprising skin ageing and/or lipodystrophy.

13. Use according to claim 12, wherein the HMG-CoA reductase inhibitor is a molecule of the family of statins or one of the physiologically acceptable salts thereof.

14. Use according to claim 13, wherein the HMG-CoA reductase inhibitor is a water-soluble statin.

15. Use according to claim 13, wherein the HMG-CoA reductase inhibitor is a lipid-soluble statin.

16. Use according to claim 13, wherein the HMG-CoA reductase inhibitor is selected from the group comprising atorvastatin, simvastatin, pravastatin, rivastatin, mevastatin (or compactin), fluindostatin, velostatin, fluvastatin, dalvastatin, cerivastatin, pentostatin, rosuvastatin, lovastatin, pitavastatin, or one of the physiologically acceptable salts thereof.

17. Use according to any one of claim 12 to 16, wherein the farnesylpyrophosphate synthase inhibitor is selected from the group comprising a molecule of the family of aminobiphosphonates (NBPs) or one of the physiologically acceptable salts thereof.

18. Use according to claim 17, wherein the aminobiphosphonate can be selected among:
- alendronic acid or the ionic form thereof, alendronate;
- clodronic acid or the ionic form thereof, clodronate;
- etidronic acid or the ionic form thereof, etidronate;
- ibandronic acid or the ionic form thereof, ibandronate;
- medronic acid or the ionic form thereof, medronate;
- neridronic acid or the ionic form thereof, neridronate;
- olpadronic acid or the ionic form thereof, olpadronate;
- pamidronic acid or the ionic form thereof, pamidronate;
- risedronic acid or the ionic form thereof, risedronate;
- tiludronic acid or the ionic form thereof, tiludronate;
- zoledronic acid or the ionic form thereof, zoledronate;
- 4-N,N-dimethylaminomethane diphosphonic acid or the ionic form thereof, dimethylaminomethanediphosphonate;
- α-amino-(4-hydroxybenzylidene) diphosphonate.

19. Use according to any one of claim 12 to 17, wherein the farnesylpyrophosphate synthase inhibitor is zoledronic acid or the ionic form thereof, zoledronate.

20. Use according to any one of claims 12 to 19 wherein the quantity of farnesylpyrophosphate synthase inhibitor is from 0.001 to 0.050 percent by weight of the dermatological composition.

21. Use according to any one of claim 12 to 20, wherein the quantity of HMG-CoA reductase inhibitor is from 0.010 to 0.100 percent by weight of the dermatological composition.

22. Use according to any one of claim 12 to 21, wherein the sum of the quantity of HMG-CoA reductase inhibitor and quantity of farnesylpyrophosphate synthase inhibitor is from 0.011 to 0.150 percent by weight of the dermatological composition.

23. Use according to any one of claim 12 to 21, wherein the cosmetic composition comprises at least one of the compounds selected from the group comprising a surfactant, thickening agent, gelling agent, preservative, humectant, emulsifier, perfume, silicone, chelating agent, antioxidant, cosmetic dye, fungicide, antibacterial agent, film-forming agent, stabilizer, buffering agent, UV filter, binder and emulsion stabilizer.

24. Use as claimed in any of claims 12 to 23, wherein the cosmetic composition is in the form of a cream, aerosol, gel, ointment, powder, foam, milk, food supplement or orally administrable product.
